(19) Europäisches Patentamt
European Patent Office
Office européen des brevets

(11) Publication number: **0 530 642 A1**

(12) **EUROPEAN PATENT APPLICATION**

(21) Application number: **92114448.1**

(22) Date of filing: **25.08.92**

(51) Int. Cl.[5]: **C07D 405/12**, A01N 43/20, C07D 409/12, C07D 513/04

(30) Priority: **04.09.91 CH 2602/91**

(43) Date of publication of application: **10.03.93 Bulletin 93/10**

(84) Designated Contracting States: **PT**

(71) Applicant: **CIBA-GEIGY AG**
**Klybeckstrasse 141**
**CH-4002 Basel(CH)**

(72) Inventor: **Brunner, Hans-Georg, Dr.**
**Wannenstrasse 14**
**CH-4415 Lausen(CH)**
Inventor: **Moser, Hans, Dr.**
**Hauptstrasse 67B**
**W-4312 Magden(CH)**
Inventor: **Pissiotas, Georg, Dr.**
**Am Sonnenrain 71**
**W-7850 Lörrach(DE)**

(54) **Herbicidal oxetane and thiaetane derivatives.**

(57) Oxetane and thiaetane derivatives of the formula I

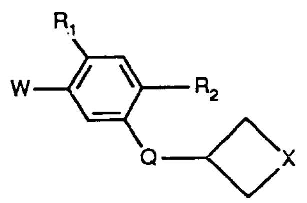
(I),

in which
Q is oxygen, sulfur, -COO-, -Z-CH($R_3$)-COO-,

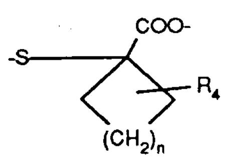
,

or CO-$Y_4$-CH($R_{10}$)-COO-;
W is

EP 0 530 642 A1

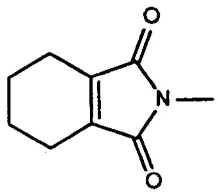 (W1), 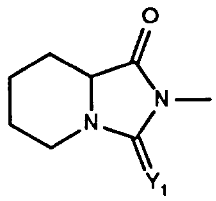 (W2), 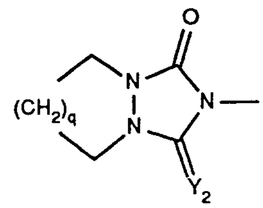 (W3),

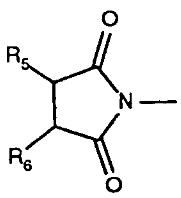 (W4), 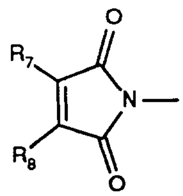 (W5), 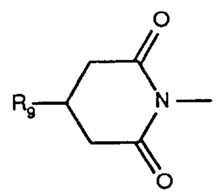 (W6),

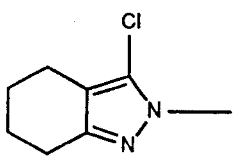 (W7), 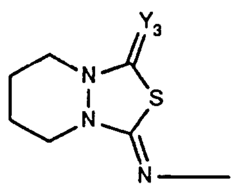 (W8) or 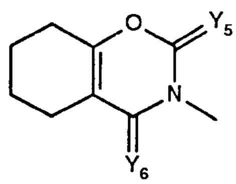 (W9) ;

and $R_1$ is hydrogen or fluorine; $R_2$ is halogen or cyano; $R_3$ and $R_{10}$ independently of one another are hydrogen or $C_1$-$C_3$alkyl; $R_4$ is hydrogen, fluorine, chlorine, bromine, $C_1$-$C_4$alkyl or trifluoromethyl; $R_5$ and $R_7$ independently of one another are $C_1$-$C_4$alkyl; $R_6$ and $R_8$ independently of one another are hydrogen or $C_1$-$C_4$alkyl; $R_9$ is hydrogen, $C_1$-$C_6$alkyl, $C_3$-$C_7$cycloalkyl, $C_1$-$C_4$alkoxy-$C_1$-$C_4$alkyl or $C_1$-$C_7$haloalkyl; X is oxygen or sulfur; $Y_1$, $Y_2$, $Y_3$, $Y_4$, $Y_5$ and $Y_6$ independently of one another are oxygen or sulfur; Z is oxygen or sulfur; n is 0; 1, 2, 3 or 4; and q is 1 or 2, have good selective herbicidal properties when used pre- or post-emergence.

The present invention relates to novel herbicidally active oxetane and thiaetane derivatives, to processes for their preparation, to the compositions comprising them as active ingredients, and to their use for controlling weeds, mainly selectively in crops of useful plants.

Herbicidally active sulfonylureas which comprise oxetane and thiaetane groups are known, for example, from EP-A-0 496 701. Novel herbicidally active oxetane and thiaetane derivatives have now been found.

The oxetane and thiaetane derivatives according to the invention are those of the formula I

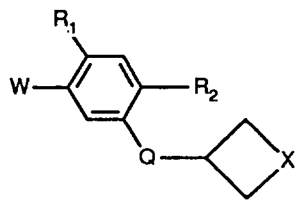 (I),

in which

Q is oxygen, sulfur, -COO-, -Z-CH($R_3$)-COO-,

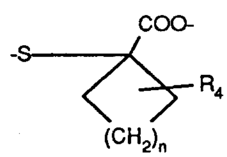 ,

or CO-$Y_4$-CH($R_{10}$)-COO-;

W is

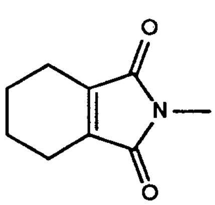 ($W_1$), 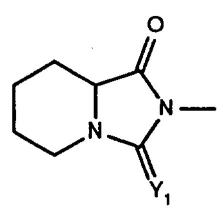 ($W_2$), 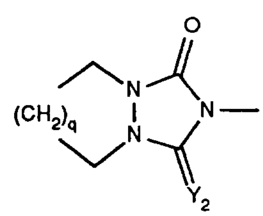 ($W_3$),

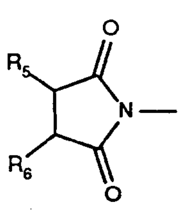 ($W_4$), 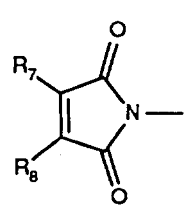 ($W_5$), 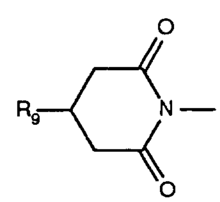 ($W_6$),

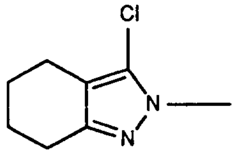 ($W_7$), 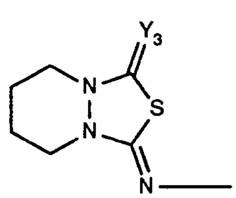 ($W_8$) or 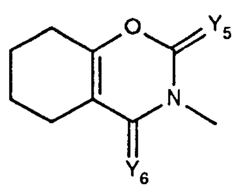 ($W_9$) ;

and $R_1$ is hydrogen or fluorine; $R_2$ is halogen or cyano; $R_3$ and $R_{10}$ independently of one another are hydrogen or $C_1$-$C_3$alkyl; $R_4$ is hydrogen, fluorine, chlorine, bromine, $C_1$-$C_4$alkyl or trifluoromethyl; $R_5$ and

$R_7$ independently of one another are $C_1$-$C_4$alkyl; $R_6$ and $R_8$ independently of one another are hydrogen or $C_1$-$C_4$alkyl; $R_9$ is hydrogen, $C_1$-$C_6$alkyl, $C_3$-$C_7$cycloalkyl, $C_1$-$C_4$alkoxy-$C_1$-$C_4$alkyl or $C_1$-$C_7$haloalkyl; X is oxygen or sulfur; $Y_1$, $Y_2$, $Y_3$, $Y_4$, $Y_5$ and $Y_6$ independently of one another are oxygen or sulfur; Z is oxygen or sulfur; n is 0; 1, 2, 3 or 4; and q is 1 or 2.

The alkyl groups which appear in the definitions of the substituents can be straight-chain or branched and are, for example, methyl, ethyl, n-propyl, iso-propyl, n-butyl, sec-butyl, iso-butyl, tert-butyl, pentyl or the isomeric pentyls, hexyl or the isomeric hexyls.

Haloalkyl is, for example, fluoromethyl, difluoromethyl, trifluoromethyl, chloromethyl, dichloromethyl, trichloromethyl, 2,2,2-trifluoroethyl, 2-fluoroethyl, 2-chloroethyl, 2,2,2-trichloroethyl or 7-chloroheptyl; preferably trichloromethyl, difluorochloromethyl, trifluoromethyl and dichlorofluoromethyl.

Alkoxy is, for example, methoxy, ethoxy, propyloxy, i-propyloxy, n-butyloxy, iso-butyloxy, sec-butyloxy and tert-butyloxy; preferably methoxy and ethoxy.

Cycloalkyl is, for example, cyclopropyl, cyclobutyl, cyclopentyl, cyclohexyl or cycloheptyl.

Halogen in the above definitions is to be understood as meaning fluorine, chlorine, bromine and iodine, preferably fluorine, chlorine and bromine.

Preferred compounds of the formula I are those in which

a) $R_1$ represents fluorine;
b) $R_2$ represents chlorine;
c) W is $W_1$, $W_6$ or $W_8$, $R_9$ preferably being trifluoromethyl;
d) $Y_1$, $Y_2$ and $Y_3$ are oxygen;
e) Q is the group

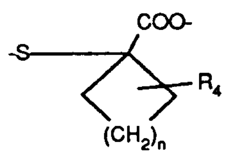

and $R_4$ is hydrogen and n is 0;
f) Q is -COO- and X is oxygen, $R_1$ preferably being fluorine and $R_2$ preferably chlorine. From amongst these compounds, those in which W is $W_8$ and $Y_3$ is oxygen are particularly preferred;
g) Q is the group -Z-CH($R_3$)-COO- or
h) Q is the group CO-$Y_4$-CH($R_{10}$)-COO-.

Individual compounds from the scope of the formula I which must be mentioned are: oxetan-3'-yl 2-chloro-4-fluoro-5(1,2-cyclohex-1-enedicarboximido)benzoate; N-[4'-chloro-2'-fluoro-5'(3''-oxetanyl)-oxyphenyl]-3,4,5,6-tetrahydrophthalimide, and 9-(4-chloro-2-fluoro-5-oxetanyloxycarbonylphenylimino)-8-thia-1,6-diazabicyclo[4.3.0]nonan-7-one, which is especially preferred.

The compounds of the formula I in which W is $W_1$, $W_4$, $W_5$, $W_6$ or $W_7$ are prepared by:

a) in the case of compounds of the formula I in which Q is -COO-, reacting a compound of the formula IIa

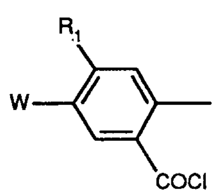
(IIa)

in which $R_1$ and $R_2$ are as defined in formula I and W is $W_1$, $W_4$, $W_5$, $W_6$ or $W_7$, with a compound of the formula IIIa

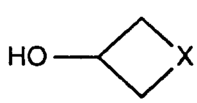
(IIIa)

in which X is as defined under formula I, in the presence of a base;

b) in the case of compounds of the formula I in which Q is oxygen or sulfur, reacting a compound of the formula IIb

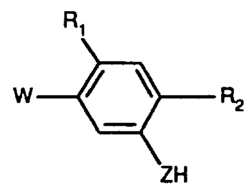
(IIb)

in which Z, $R_1$ and $R_2$ are as defined in formula I and W is $W_1$, $W_4$, $W_5$, $W_6$ or $W_7$, with a compound of the formula IIIb

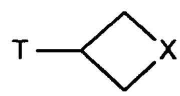
(IIIb)

in which X is as defined in formula I and T is tosyl or mesyl, preferably mesyl, in the presence of a base;

c) in the case of compounds of the formula I in which Q is -Z-CH($R_3$)-COO-, reacting a compound of the formula IIc

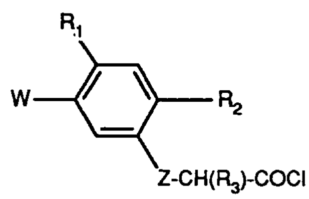
(IIc)

in which Z, $R_1$, $R_2$ and $R_3$ are as defined in formula I and W is $W_1$, $W_4$, $W_5$, $W_6$ or $W_7$, with a compound of the formula IIIa

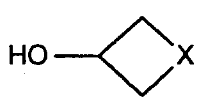
(IIIa)

in which X is as defined in formula I, in the presence of a base, or a compound of the formula IIb

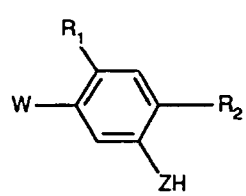
(IIb)

in which Z, $R_1$ and $R_2$ are as defined in formula I and W is $W_1$, $W_4$, $W_5$, $W_6$ and $W_7$, with a compound of the formula IIIc

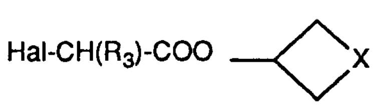
(IIIc)

in which X and $R_3$ are as defined for formula I and Hal is fluorine, chlorine or bromine, in the presence of a base;

d) in the case of compounds of the formula I in which Q is the group

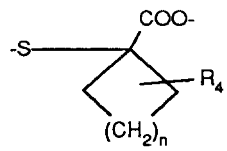 ,

reacting a compound of the formula IId

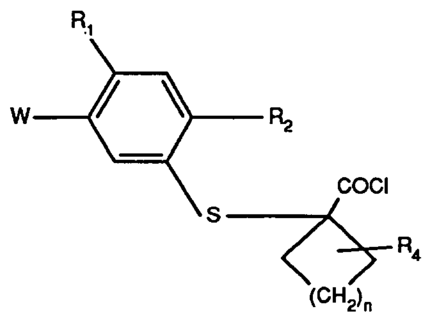

(IId)

in which $R_1$, $R_2$, $R_4$ and n are as defined in formula I and W is $W_1$, $W_4$, $W_5$, $W_6$ or $W_7$, with a compound of the formula IIIa

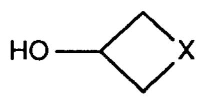

(IIIa)

in which X is as defined in formula I, in the presence of a base;

e) in the case of compounds of the formula I in which Q is the group CO-$Y_4$-CH($R_{10}$)-COO-, reacting a compound of the formula IIe

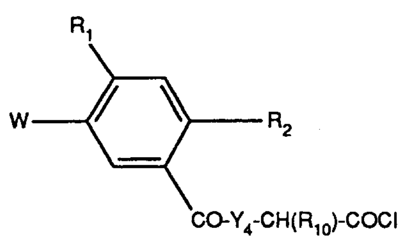

(IIe)

in which $R_1$, $R_2$, $R_3$ and $Y_4$ are as defined in formula I and W is $W_1$, $W_4$, $W_5$, $W_6$ or $W_7$, with a compound of the formula IIIa

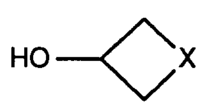

(IIIa)

in which X is as defined in formula I, in the presence of a base.

The compounds of the formulae IIIa, IIIb and IIIc are known or can be prepared analogously to known processes (see, for example, B. Lamm et al., Acta Chem. Scand. 28, 701 (1974) or J. Org. Chem. 48, 2953-2956(1983)).

The compounds of the formulae IIa,, IIb, IIc, IId and IIe in which W is $W_7$ are known, for example, from EP-A-0 370 995, EP-A-0 206 435 or EP-A-0 138 527 or can be prepared analogously to known processes.

The compounds of the formulae IIa, IIb, IIc, IId and IIe in which W is $W_1$, $W_4$, $W_5$ or $W_6$ can be prepared by reacting the respective anhydrides of the radicals $W_1$ (tetrahydrophthalic anhydride), $W_4$ -(maleic anhydride), $W_5$ (succinic anhydride) and $W_6$ (glutaric anhydride) with amines of the formulae IVa, IVb, IVc, IVd and IVe

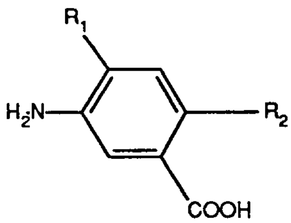 (IVa), 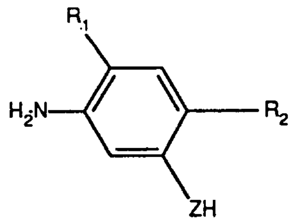 (IVb),

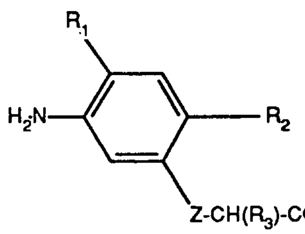 (IVc), 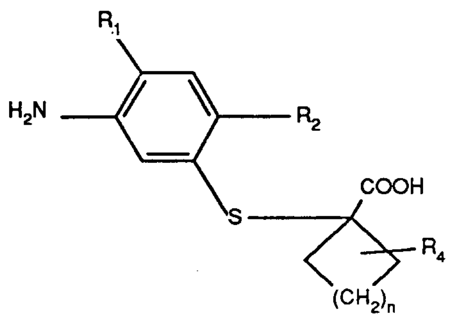 (IVd),

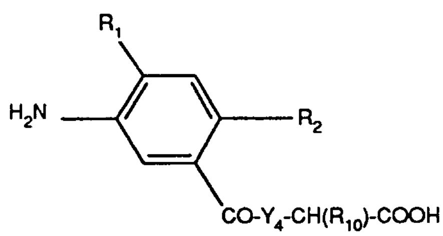 (IVe),

in which $R_1$, $R_2$, $R_3$, $R_4$, $R_{10}$, $Y_4$, Z and n are as defined in formula I. For example, compounds of the formula IId in which W is $W_6$ can be prepared by reacting a glutaric anhydride of the formula V in which $R_9$ is as defined in formula I with an amine of the formula IVd in which $R_1$, $R_2$, $R_4$ and n are as defined in formula I to give a glutamonoanilide of the formula VI, and the resulting monoamide of the formula VI is subsequently cyclised with a condensing agent to give a compound of the formula VIa, which is reacted with a chlorinating agent, for example phosphorus trichloride, phosphorus pentachloride, thionyl chloride or phosphorus oxychloride, to give the compounds of the formula IId.

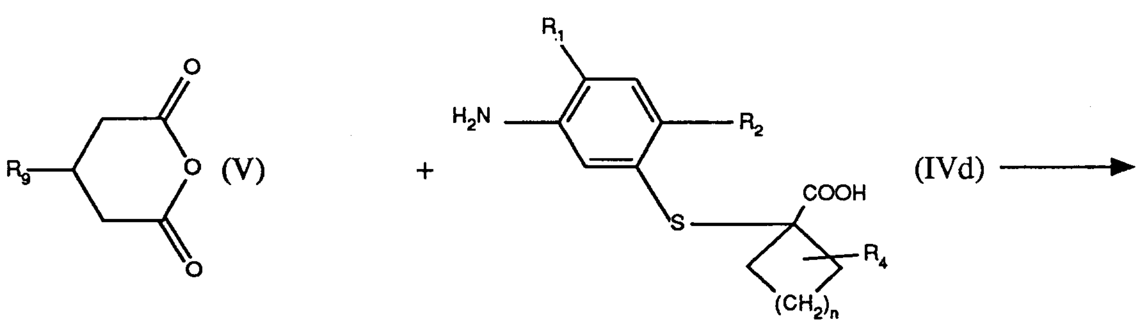
(V) + (IVd)

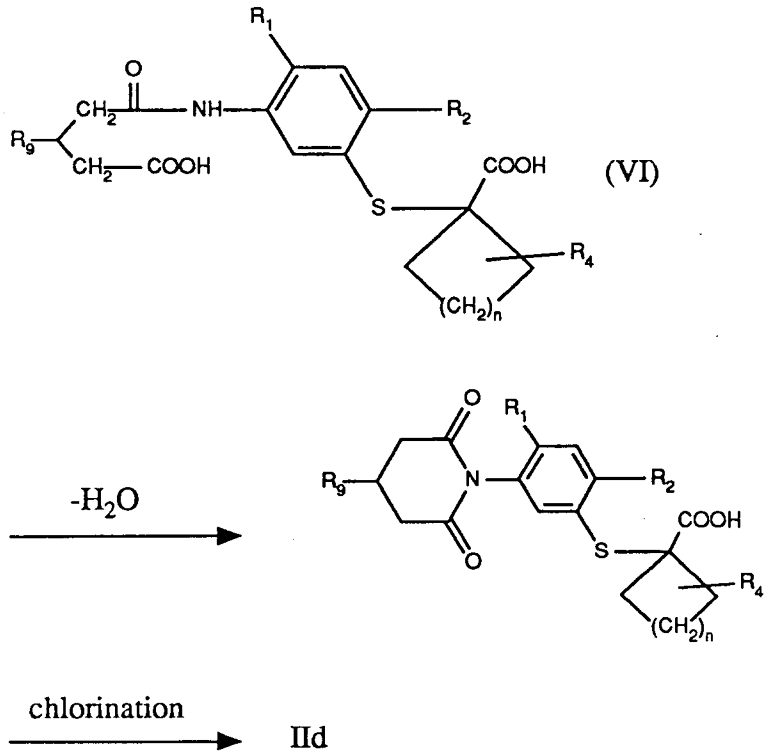


(VI)

(VIa)

IId

The above reaction is advantageously carried out in an inert organic solvent. The reaction temperature is generally between room temperature and the boiling point of the reaction mixture, the reaction is preferably refluxed. The condensation reaction can be accelerated by adding condensation catalyst and removing the water being formed. The same effect is achieved by adding dehydrating agents, for example sulfuric acid. Suitable solvents are, in particular, higher-boiling hydrocarbons, lower alkanecarboxylic acids and their esters and amides, higher-boiling ketones and ethers. Examples of suitable solvents are: benzene, toluene, xylene, dimethylformamide, dimethylacetamide, acetic acid, ethyl acetate, diisopropyl ether, ethylene glycol dimethyl ether, tetrahydrofuran or 2-butanone.

Examples of suitable catalysts are: p-toluenesulfonic acid, benzoic acid, 4-dimethylaminopyridine, sulfuric acid, hydrogen chloride or naphthalenesulfonic acid. The compounds of the formulae IIa to IIb in which W is $W_1$, $W_4$ or $W_5$ can be prepared analogously.

The compounds of the formula V or the corresponding anhydrides of the radicals $W_1$, $W_4$, and $W_5$ as well as the compounds of the formula VI are known or can be prepared analogously to known processes.

Various routes are described in the literature to synthesise anhydrides of the formula V and the anhydrides of the radicals $W_1$, $W_4$ and $W_5$.

A suitable reaction sequence for the preparation of the compounds of the formula V proves to be the sequence outlined in diagram 1, which is based on very simple, generally available components (aldehyde and cyanoacetate):

Diagram 1

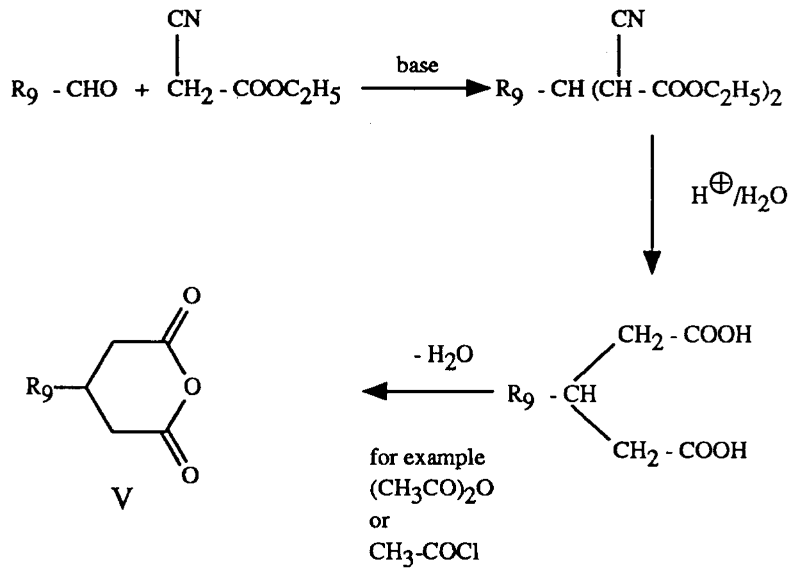


The process according to diagram 1 is described in greater detail, inter alia, in the following references: J. Chem. Soc. 117 (1920) 1465; J. Chem. Soc. 123 (1923), 3131; J. Chem. Soc. 1952, 4785; Rec. Trav. Chim. Pays-Bas 84 (1965), 1183 and J. Ind. Chem. Soc. 13 (1936), 322.

Another process variant for the preparation of the glutaric anhydrides of the formula V is based on a malonate synthesis and compiled in diagram 2:

Diagram 2

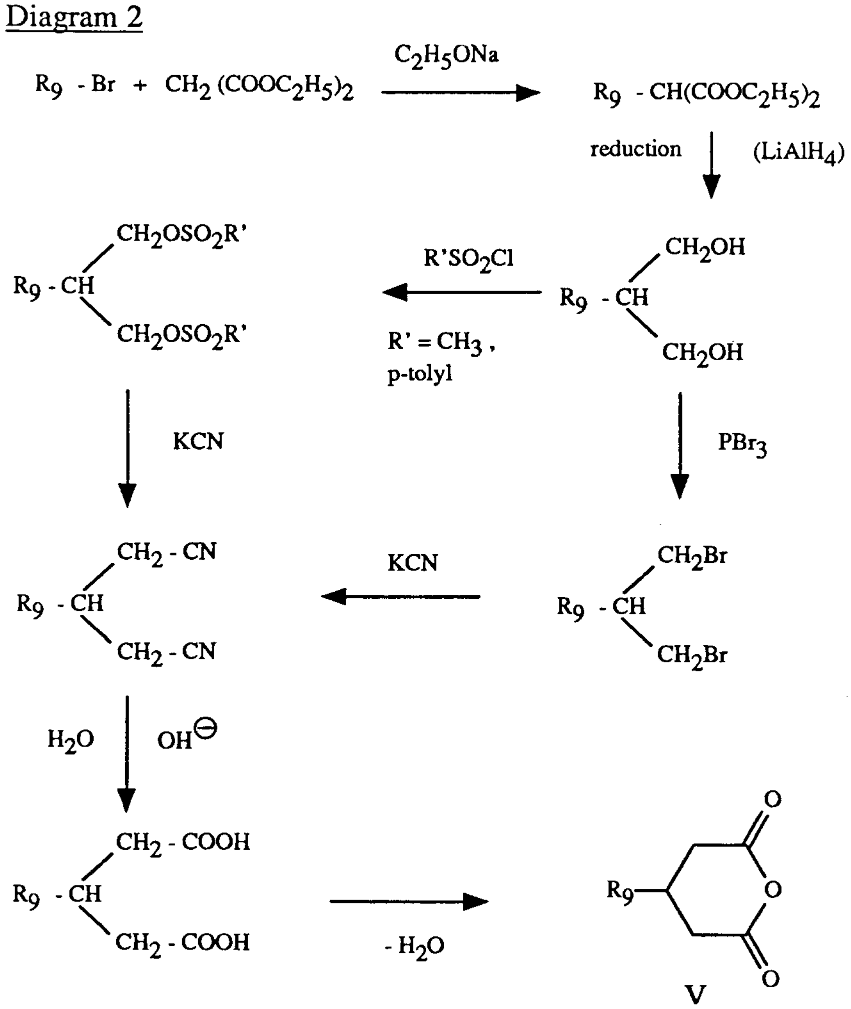


V

The anhydrides of the radicals $W_1$, $W_4$ and $W_5$ can be prepared analogously (see, for example, USP 4 804 400).

The process outlined in diagram 2 is known, inter alia, from the following references:
Chem. Soc. Perkin Trans. I, 1978, 1636; J. Am. Chem. Soc. 95 (1973), 7437; Org. Prep. Proc. Int. 7, (1975), 283.

The anilines of the formulae IVa to IVe are known or can be prepared by known methods, for example by reduction of the corresponding nitro compounds by catalytic methods (for example $H_2$/Pd/C, or $H_2$/Pt) or, as described below for the preparation of the compound of the formula IVd in which n is other than 0, by reacting a thiophenol of the formula VII with a compound of the formula VIII in the presence of a base as in the following diagram:

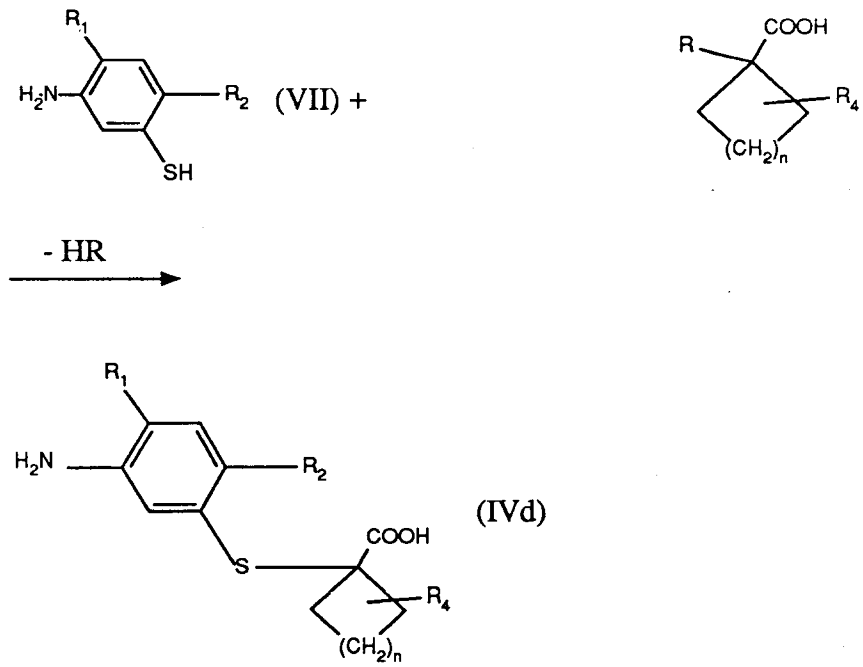


(VII) + (VIII)

(IVd)

In the above diagram, R is a group which can be exchanged under the reaction conditions, for example halogen, preferably chlorine, bromine or iodine, or a phenylsulfonyl radical which is optionally alkylated or halogenated in the phenyl ring, the substituents $R_1$, $R_2$ and $R_4$ being defined as in formula I and n being 1,2,3 or 4. The preparation of the compounds of the formulae IVa, IVb, IVc and IVe can be carried out analogously to the process described for the compound of the formula IVd.

Compounds of the formula IVd in which n is 0 can be prepared by reacting a compound of the formula VII with a compound of the formula XXXI

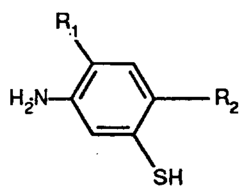

(VII) +

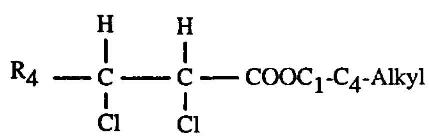

(XXXI)

in the presence of a base to give the compound of the formula XXXII

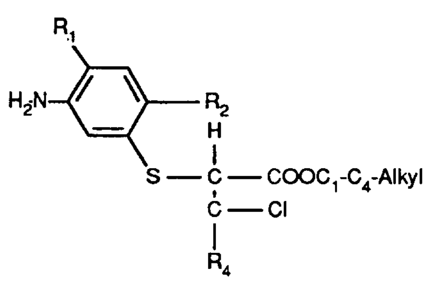

(XXXII)

in which $R_1$, $R_2$ and $R_4$ are as defined in formula I, cyclising the product in the presence of a base and subsequently eliminating the $C_1$-$C_4$alkyl group by hydrolysis under basic conditions.

Bases which are suitable for these reactions are oxides, hydrides, hydroxides, carbonates, carboxylates or alcoholates of an alkali metal or alkaline earth metal, trialkylamines or pyridine bases. Bases which have proved particularly suitable are sodium methylate, sodium ethylate, sodium hydroxide, potassium hydroxide, sodium carbonate or potassium carbonate. Examples of solvents which are suitable for these reactions are compounds from the group of the open-chain or cyclic ethers such as dioxane, tetrahydrofuran, diethyl

ether, diisopropyl ether, dimethoxymethane or 1,2-dimethoxyethane, from the groups of the alcohols such as methanol, ethanol or isopropanol, or from the group of the ketones such as acetone, methyl ethyl ketone or methyl isobutyl ketone. The reactions are generally carried out at temperatures from room temperature to the boiling point of the solvent.

The thiophenols of the formula VII are disclosed in EP-A-0 259 265. The compounds of the formula VIII are known or can be prepared analogously to known methods (see, for example, J. Org. Chem. **54**, 6096-6100 (1989)).

The compounds of the formula I in which W is $W_2$ and $Y_1$ is oxygen are prepared by condensing an isocyanate of the formula IX

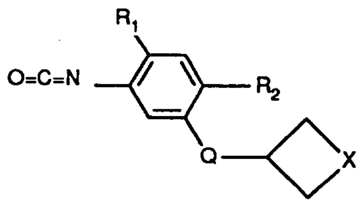

(IX)

in which $R_1$, $R_2$, Q and X are as defined in formula I, with a piperidine of the formula X

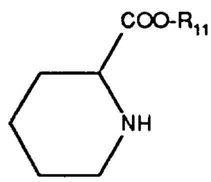

(X)

in which $R_{11}$ is $C_1$-$C_6$alkyl or benzyl, in an organic solvent in the presence of a base at a temperature of 0 to +150°C to give the compound of the formula XI

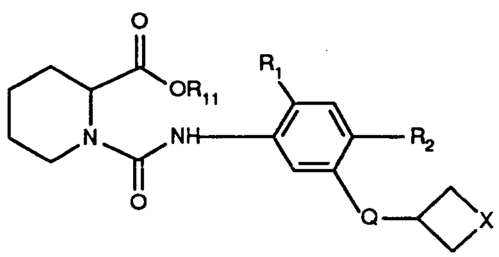

(XI)

in which $R_1$, $R_2$, X and Q are as defined in formala I and $R_{11}$ is as defined in formula X, and the compound of the formula XI is subsequently cyclised under acid conditions at a temperature of +50 to +150°C to give the compound of the formula I. Such reactions are described, for example, in EP-A-0 211 805.

The compounds of the formula I in which W is $W_2$ and $Y_1$ is sulfur are prepared by condensing an isothiocyanate of the formula XII

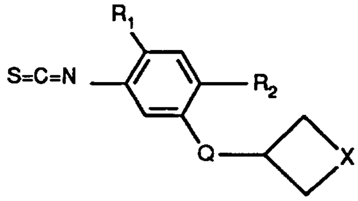

(XII)

in which $R_1$, $R_2$, X and Q are as defined in formula I with a piperidine of the formula X

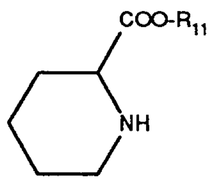

(X)

in which $R_{11}$ is $C_1$-$C_6$alkyl or benzyl, in an organic solvent in the presence of a base at a temperature from 0 to +150 ° C to give the compound of the formula XIII

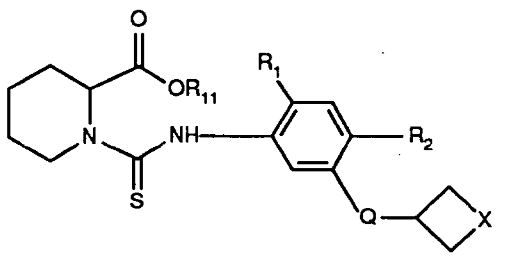

(XIII)

in which $R_1$, $R_2$, X and Q are as defined in formula I and $R_{11}$ is as defined in formula X, and the compound of the formula XIII is subsequently cyclised under acid conditions at a temperature from +50 to +150 ° C to give the compound of the formula I. Such reactions are also described in EP-A-0 211 805.

The compounds of the formula I in which W is $W_3$ and $Y_2$ is sulfur can be prepared by condensing an isocyanate of the formula IX

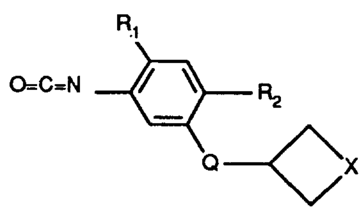

(IX)

in which $R_1$, $R_2$, Q and X are as defined in formula I, with a compound of the formula XIX

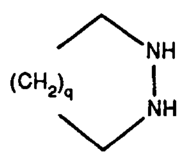

(XIX)

in which q is as defined in formula I, in an organic solvent in the presence of a base at a temperature from 0 to +150 ° C, and the resulting compound of the formula XXV

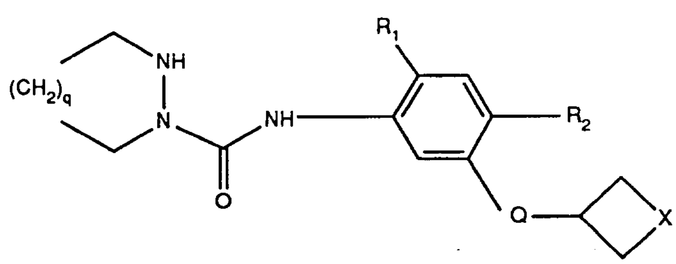

(XXV)

in which $R_1$, $R_2$, q, X and Q have the abovementioned meaning is subsequently reacted with thiophosgene in the presence of an inorganic base at a temperature from 0 to +150 °C. Such reactions are described, for example, in EP-A-0 211 805.

The compounds of the formula I in which W is $W_3$ and $Y_2$ is oxygen can be prepared by condensing an isocyanate of the formula IX

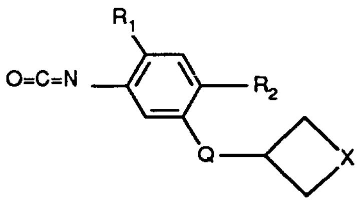

(IX)

in which $R_1$, $R_2$, X and Q have the abovementioned meaning, with a compound of the formula XXVI

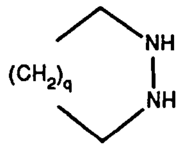

(XXVI)

in which q is as defined in formula I, in an organic solvent in the presence of a base at a temperature from 0 to +150 °C, subseqently reacting the resulting compound of the formula XXVII

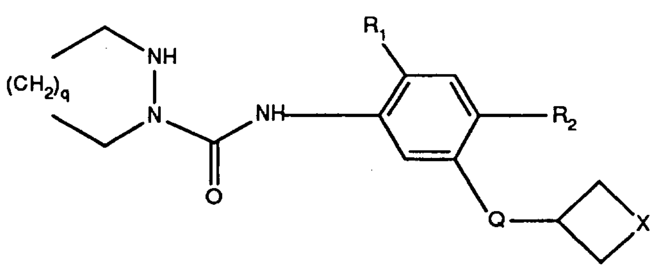

(XXVII)

in which $R_1$, $R_2$, q, X and Q have the abovementioned meaning, with a chloroformic acid ester of the formula XXVIII

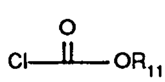

(XXVIII)

in which $R_{11}$ is $C_1$-$C_6$alkyl or benzyl, in the presence of a base at a temperature from 0 to +150 °C, to give a compound of the formula XXIX

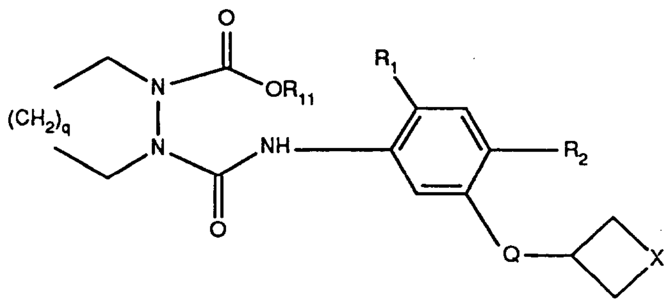

(XXIX)

in which $R_1$, $R_2$, $R_{11}$, q, X and Q have the abovementioned meaning, and these compounds of the formula XXIX are cyclised in an inert solvent in the presence of a base at a temperature from +50 to +180 °C to give the compounds of the formulae IIa to IIe. Such reactions are also described in EP-A-0 211 805.

The compounds of the formula I in which W is $W_8$ are prepared by converting an isothiocyanate of the formula XII

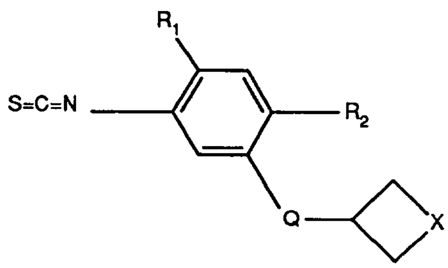

(XII)

in which $R_1$, $R_2$, X and Q are as defined in formula I, with a hexahydropyridazine of the formula XVIII

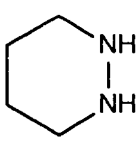

(XVIII)

into the compound of the formula XIX

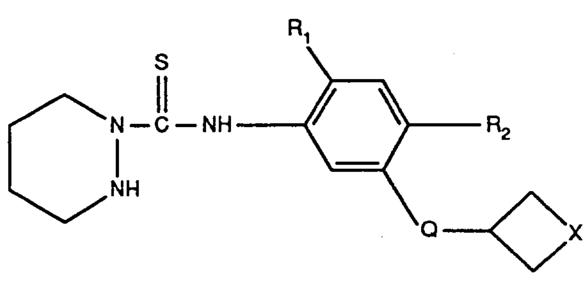

(XIX)

in which $R_1$, $R_2$, X and Q have the abovementioned meanings, and these compounds of the formula XIX are subsequently reacted with a compound of the formula XXX

$CY_3Cl_2$ (XXX),

in which $Y_3$ is oxygen or sulfur, in the presence of a base.

The compounds of the formula I in which W is $W_9$ can be prepared by reacting a 2-hydroxycyclohex-1-enecarboxanilide or -thiocarboxanilide of the formula XXXI

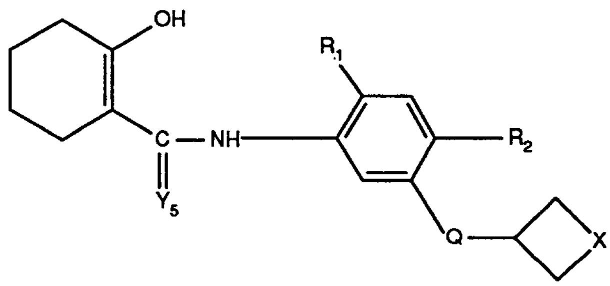

(XXXI)

in which Q, X, $R_1$, $R_2$ and $Y_5$ are as defined in formula I, with a reactive carbonic acid derivative or thiocarbonic acid derivative of the formula XXXII

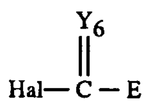

(XXXII)

in which $Y_6$ is oxygen or sulfur, Hal is chlorine or bromine and E is chlorine, bromine or $C_1$-$C_4$alkoxy, resulting in cyclisation to give a compound of the formula I.

Cyclisation takes place in the presence of the equimolar amount of an organic or inorganic base in an inert solvent or diluent at a temperature from room temperature to the boiling point of the reaction mixture.

Suitable reactive carbonic acid esters, or thiocarbonic acid esters, are, for example, phosgene, thiophosgene, ethyl chloroformate and methyl chloroformate.

Solvents and diluents which are suitable are acetone, methyl ethyl ketone, dioxane or tetrahydrofuran, acetonitrile, toluene or chloroform.

The intermediates of the formula XXXI are known or can be prepared analogously to known processes. To prepare the compounds of the formula XXXIa or XXXIb, morpholinocyclohexene is reacted with an isocyanate of the formula IX, or isothiocyanate of the formula XII, respectively, and the resulting 2-morpholinocyclohex-1-enecarboxanilide XXXIVa, or 2-morpholinocyclohex-1-enethiocarboxanilide of the formula XXXIVb, respectively, is subsequently hydrolysed in aqueous acidic solution following the two diagrams below:

<u>Diagram 3:</u>

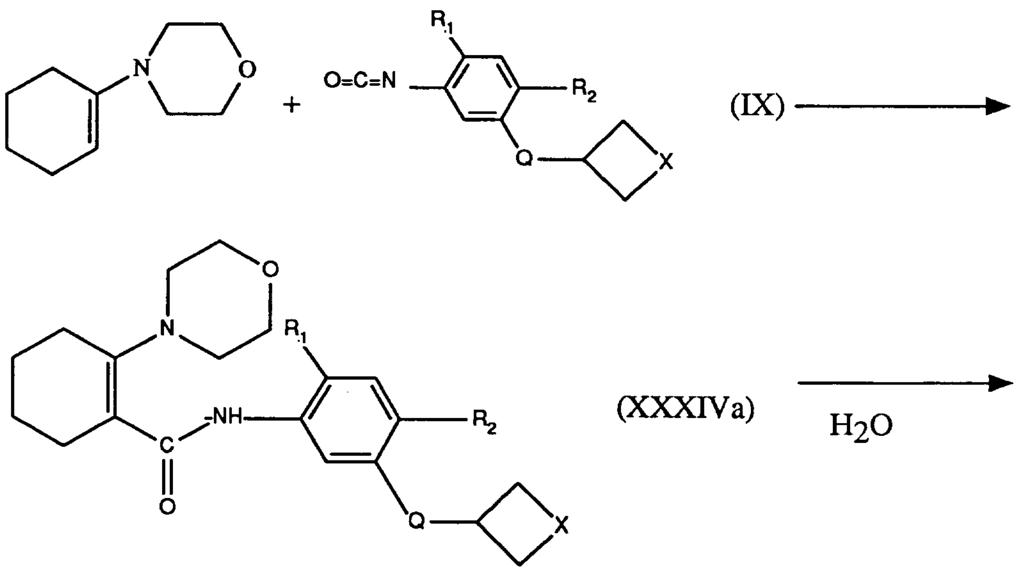


(IX)

(XXXIVa)

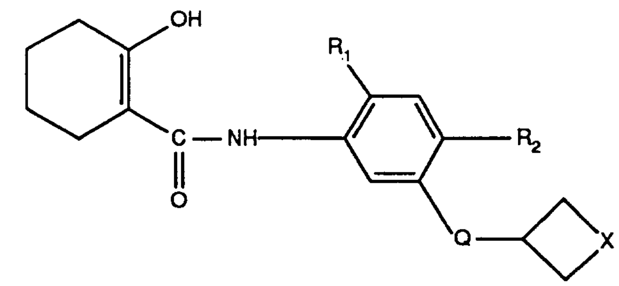

(XXXIa)

Diagram 4:

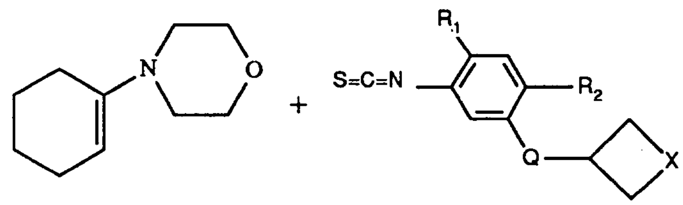

(XII)

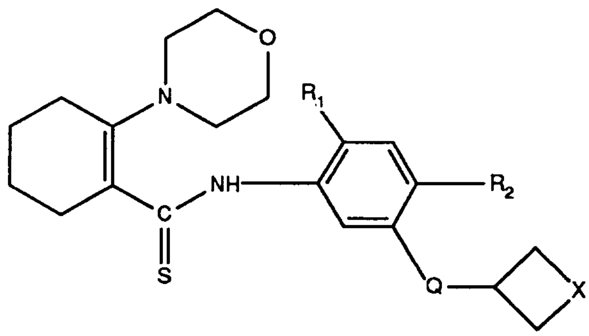

(XXXIVb)

$H_2O$

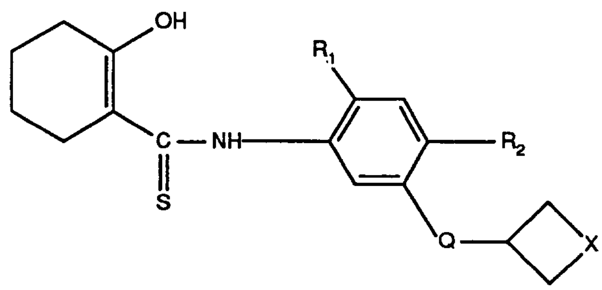

(XXXIb)

The substituents in the above formulae are as defined in formula I. 1-Morpholinocyclohex-1-ene is known.

The reaction of the isothiocyanates of the formula XII with the pyridazines of the formula XVIII is advantageously carried out in a solvent which is inert under the reaction conditions at temperatures from -5 ° C to the boiling point of the solvent, in particular 0 to +50 ° C, particularly preferably at room temperature. Solvents which are suitable for this reaction are, for example, toluene, xylene, ethyl acetate or acetontrile.

The reaction of the compound of the formula XIX with the compound of the formula XXX is advantageously carried out in a solvent which is inert under the reaction conditions at low temperatures, preferably at 0 to +50 ° C, particularly preferably at 0 to +15 ° C. Bases which are suitable for this reaction are, for example, pyridine, triethylamine or N,N-dimethylaniline. Suitable solvents are, for example, 1,2-dichloroethane, dichloromethane or toluene.

The pyridazines of the formula XVIII which are used as starting materials for the compounds of the formulae IIa to IIe according to the invention, in which W is $W_8$, are known or can be prepared analogously to processes known from the literature. The preparation of such a compound is described, for example, in Coll. Czechoslov. Chem. Commun. 33, 2087 (1968), Khimiya Geterotsiklicheskikh Soedinenii, 4, (3), 530-535 (1968), Beilstein, E III/IV 23, p. 465 and EP-A-0 304 920.

The isocyanates of the formula IX, or isothiocyanates of the formula XII, are known or can be prepared analogously to known processes.
The isocyanates, or isothiocyanates, of the formulae XII and IX are obtained in a manner known per se by reacting the corresponding amines of the formulae IVa to IVe with phosgene or thiophosgene, or with a reactive carbonic acid ester or thiocarbonic acid ester of the formula XXa

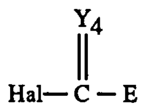
(XXa)

in which $Y_4$ is oxygen or sulfur, Hal is chlorine or bromine and E is chlorine, bromine or $C_1$-$C_4$alkoxy. Such processes are described, for example in EP-A-0 304 920.

The processes listed above can be carried out analogously to the processes known from the literature. The reaction conditions preferred in these processes, such as temperature, molar ratios of the educts, reaction procedure, solvents, reagents which may be necessary such as acids, bases, water-binding agents and the like, are known to those skilled in the art.

The compounds of the formula I are employed as pure active ingredients as obtainable from synthesis or, preferably, together with auxiliaries conventionally used in the art of formulation and they are therefore processed in a known manner to give, for example, emulsifiable concentrates, directly sprayable or dilutable solutions, dilute emulsions, wettable powders, soluble powders, dusts, granules, and also encapsulations, for example in polymeric substances. The application methods, such as spraying, atomising, dusting, wetting, scattering or pouring as well as the nature of the compositions are selected to suit the intended aims and the prevailing circumstances.

The formulations, i.e. the compositions, preparations or combinations comprising the active ingredient of the formula I and, if desired, one or more solid or liquid additives, are prepared by known methods, for example by intimately mixing and/or grinding the active ingredients with extenders, for example with solvents, carriers and, if appropriate, surface-active compounds (surfactants).

The following are possible as solvents: aromatic hydrocarbons, in particular the fractions $C_8$ to $C_{12}$, such as mixtures of alkylbenzenes, for example xylene mixtures or alkylated naphthalenes; aliphatic and cycloaliphatic nitrocarbons such as paraffins, cyclohexane or tetrahydronaphthalene; alcohols such as ethanol, propanol or butanol; glycols as well as their ethers and esters, such as propylene glycol or dipropylene glycol ether, ketones such as cyclohexanone, isophorone or diacetone alcohol, strongly polar solvents such as N-methyl-2-pyrrolidone, dimethyl sulfoxide or water; vegetable oils as well as their esters, such as rapeseed oil, castor oil or soya oil; optionally also silicone oils.

Solid carriers which are generally used, for example for dusts and dispersible powders, are ground natural minerals, such as calcite, talc, kaolin, montmorillonite or attapulgite. To improve the physical properties, it is also possible to add highly-disperse silica or highly-disperse absorptive polymers. Possible particulate adsorptive carriers for granules are either porous types, for example pumice, brick grit, sepiolite or bentonite, or non-sorptive carrrier materials, for example calcite or sand. Moreover, a large number of pregranulated materials of inorganic or organic nature can be used, such as, in particular, dolomite or comminuted plant residues.

Suitable surface-active compounds are non-ionic, cationic and/or anionic surfactants having good emulsifying, dispersing and wetting properties, depending on the nature of the active ingredient of the formula I to be formulated. Surfactants are also to be understood as meaning mixtures of surfactants.

Suitable anionic surfactants can be either so-called water-soluble soaps or water-soluble synthetic surface-active compounds.

Suitable soaps which may be mentioned are the alkali metal salts, alkaline earth metal salts or substituted or unsubstituted ammonium salts of higher fatty acids ($C_{10}C_{22}$), for example the sodium or potassium salts of oleic or stearic acid or of natural mixtures of fatty acids which can be obtained, for example, from coconut oil or tallow oil. Fatty acid methyltaurides may furthermore be mentioned.

However, so-called synthetic surfactants are used more frequently, in particular fatty alcohol sulfonates, fatty alcohol sulfates, sulfonated benzimidazole derivatives or alkylarylsulfonates.

The fatty alcohol sulfonates or fatty alcohol sulfates generally exist in the form of alkali metal salts, alkaline earth metal salts or substituted or unsubstituted ammonium salts and have an alkyl radical of 8 to 22 C atoms, alkyl also including the alkyl moiety of acyl radicals, for example the sodium or calcium salt of ligninsulfonic acid, of the dodecylsulfuric acid ester or of a fatty alcohol sulfate mixture prepared from

natural fatty acids. This group also embraces the salts of the sulfuric acid esters and sulfonic acids of fatty alcohol/ethylene oxide adducts. The sulfonated benzimidazole derivatives preferably contain 2 sulfonyl groups and one fatty acid radical having 8-22 C atoms. Examples of alkylarylsulfonates are the sodium, calcium or triethanolamine salts of dodecylbenzenesulfonic acid, or dibutylnaphthalenesulfonic acid, or of a naphthalenesulfonic acid/formaldehyde condensation product.

Other suitable compounds are corresponding phosphates, for example salts of the phosphoric acid ester of a p-nonylphenol/(4-14) ethylene oxide adduct, or phospholipids.

Suitable non-ionic surfactants are mainly polyglycol ether derivatives of aliphatic or cycloaliphatic alcohols, saturated or unsaturated fatty acids and alkylphenols, which can contain 3 to 30 glycol ether groups and 8 to 20 carbon atoms in the (aliphatic) hydrocarbon radical and 6 to 18 carbon atoms in the alkyl radical of the alkylphenols.

Other suitable non-ionic surfactants are the water-soluble polyethylene oxide adducts with polypropylene glycol, ethylenediaminopolypropylene glycol and alkylpolypropylene glycol which have 1 to 10 carbon atoms in the alkyl chain and contain 20 to 250 ethylene glycol ether groups and 10 to 100 propylene glycol ether groups. The compounds mentioned generally contain 1 to 5 ethylene glycol units per propylene glycol unit.

Examples of non-ionic surfactants which may be mentioned are nonylphenol polyethoxyethanols, castor oil polyglycol ethers, polypropylene/polyethylene oxide adducts, tributylphenoxypolyethoxyethanol, polyethylene glycol and octylphenoxypolyethoxyethanol.

Other suitable substances are fatty acid esters of polyoxyethylene sorbitan, such as polyoxyethylene sorbitan trioleate.

The cationic surfactants are mainly quaternary ammonium salts which contain at least one alkyl radical having 8 to 22 C atoms as N substituents and which have lower halogenated or free alkyl, benzyl or lower hydroxyalkyl radicals as further substituents. The salts are preferably in the form of halides, methylsulfates or ethylsulfates, for example stearyltrimethylammonium chloride or benzyldi(2-chloroethyl)ethylammonium bromide.

The surfactants customary in the art of formulation are described, inter alia, in the following publications:

- "Mc Cutcheon's Detergents and Emulsifiers Annual", Mc Publishing Corp., Glen Rock, New Jersey, 1988.
- M. and J. Ash, "Enclyclopedia of Surfactants", Vol. I-III, Chemical Publishing Co., New York, 1980-1981.
- Dr. Helmut Stache "Tensid-Taschenbuch [Surfactant Guide]", Carl Hanser Verlag, Munich/Vienna 1981.

The pesticidal preparations generally comprise 0.1 to 99%, in particular 0.1 to 95%, of active ingredient of the formula I, 1 to 99% of a solid or liquid additive and 0 to 25%, in particular 0.1 to 25%, of a surfactant.

While concentrated compositions are more preferred as commercially available goods, the end consumer generally uses dilute composition.

The compositions can also comprise further additives such as stabilisers, for example epoxidised or unepoxidised vegetable oils (epoxidised coconut oil, rapeseed oil or soya oil),defoamers, for example silicone oil, preservatives, viscosity regulators, binders, adhesives as well as fertilisers or other active ingredients for achieving specific effects.

In particular, preferred formulations have the following composition: (% = percent by weight).

| Emulsifiable concentrates: | |
|---|---|
| Active ingredient: | 1 to 90%, preferably 5 to 20% |
| Surface-active composition: | 1 to 30%, preferably 10 to 20% |
| Liquid carrier: | 15 to 94%, preferably 70 to 85% |

| Dusts: | |
|---|---|
| Active ingredient: | 0.1 to 10%, preferably 0.1 to 1% |
| Solid carrier: | 99.9 to 90%, preferably 99.9 to 99% |

| Suspension concentrates: | |
|---|---|
| Active ingredient:<br>Water:<br>Surface-active composition: | 5 to 75%, preferably 10 to 50%<br>94 to 24%, preferably 88 to 30%<br>1 to 40%, preferably 2 to 30% |

| Wettable powders: | |
|---|---|
| Active ingredient:<br>Surface-active composition:<br>Solid carrier material: | 0.5 to 90%, preferably 1 to 80%<br>0.5 to 20%, preferably 1 to 15%<br>5 to 95%, preferably 15 to 90% |

| Granules: | |
|---|---|
| Active ingredient:<br>Solid carrier material: | 0.5 to 30%, preferably 3 to 15%<br>99.5 to 70%, preferably 97 to 85% |

The active ingredients of the formula I are generally successfully employed at application rates of 0.001 to 2 kg/ha, in particular 0.005 to 1 kg/ha. The dosage rate required for the desired action can be determined by trials. It depends on the nature of action, the development stage of the crop plant and of the weed, and on the application (location, time, method) and can be varied within a wide range through these parameters.

When used at lower application rates, the compounds of the formula I are distinguished by growth-inhibiting and herbicidal properties which make them outstandingly suitable for use in crops of useful plants, in particular in cereals, cotton, soya, oilseed rape, maize and rice.

The invention also relates to herbicidal compositions which comprise a novel active ingredient of the formula I and to methods for inhibiting plant growth.

Preparation Examples:

Example H1: Preparation of 3-oxetanyl mesylate:

25.2 ml of methane sulfochloride are added dropwise with stirring at 0 °C to a solution of 27 g of 3-oxetanol (89%) prepared by the method described in J. Org. Chem 48, 2953 (1983) and 45.2 ml of triethylamine in 800 ml of methylene chloride. The mixture is stirred for 3 hours at room temperature, the precipitated triethylamine hydrochloride is filtered off, and the filtrate is washed first with ice-water and then with saturated sodium chloride solution. The liquid residue which remains after evaporation of the methylene chloride is distilled under reduced pressure. 38.3 g of the title compound of b.p.: 79-85 °C at a pressure of 1.4 mbar are obtained.

Example H2: Preparation of 4-chloro-2-fluoro-5-(3'-oxetanyl)oxyaniline:

36.6 g of 4-chloro-2-fluoro-5-hydroxyaniline are dissolved in 700 ml of DMSO, and 15.5 g of pulverulent KOH (90%) are added to the solution, with stirring. During this process, the temperature rises to 35 °C. After 2 hours, 34.5 g of 3-oxetanyl mesylate are added, and the reaction mixture, which is dark, is stirred overnight at a bath temperature of 80 °C. When cold, the reaction solution is poured onto ice and extracted with ethyl acetate. The ethyl acetate fractions are combined, washed twice using saturated sodium chloride solution, dried over sodium sulfate and evaporated under reduced pressure. The dark oil which remains is chromatographed over a silica gel column using hexane/ethyl acetate 2:1. This gives a main fraction and a mixed fraction. These fractions are evaporated separately and recrystallised from carbon tetrachloride or diisopropyl ether. 11.3 g of the title compound of m.p. 67-70 °C are isolated from the main fraction, and 8.6 g of m.p. 89-91 °C from the mixed fraction.

Example H3: Preparation of N-[4'-chloro-2'-fluoro-5'-(3''-oxetanyl)oxyphenyl]-3-trifluoromethylglutarmonoamide:

10.3 g of the aniline described in the above example are dissolved in 90 ml of toluene. 8.6 g of 3-trifluoromethylglutaric anhydride are added, with stirring. Within a short time, a thick precipitate is obtained. After 1 hour, this precipitate is filtered off using a suction filter, washed with cold toluene and dried. 16.4 g of N-[4'-chloro-2'-fluoro-5'-(3''-oxetanyl)oxyphenyl]-3-trifluoromethylglutarmonoamide of m.p. 198-201 °C are obtained.

Example H4: Preparation of 1-[4'-chloro-2'-fluoro-5'-(3''-oxetanyl)oxyphenyl]-4-trifluoromethylpiperidine-2,6-dione (Compound 1.046):

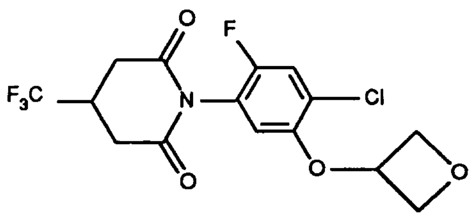

(1.046)

13.4 g of the above described glutarmonoamide and 2.75 g of sodium acetate are stirred for half an hour in 104 ml of acetic anhydride at a bath temperature of 80 °C. After the mixture has cooled to room temperature, the resulting acetic acid and the excess anhydride are removed by distillation under a waterpump vacuum. The solid residue is partitioned between ethyl acetate and ice-water, and the organic phase is washed with 10% sodium bicarbonate solution and with saturated sodium chloride solution, dried over sodium sulfate and evaporated in vacuo. Recrystallisation of the residue from ethylene chloride gives 9.2 g of the title compound No. 1.046 of m.p. 190-192 °C.

Example H5: Preparation of N-[4'-chloro-2'-fluoro-5'-(3''-oxetanyl)oxyphenyl]-3,4,5,6-tetrahydrophthalimide (Compound 1.005):

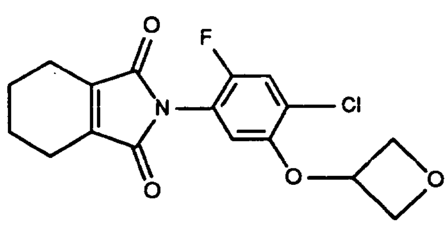

(1.005)

6.6 g of the aniline described in Example 2 and 7 g of 3,4,5,6-tetrahydrophthalic anhydride are stirred in 120 ml of glacial acetic acid, first for one hour at a bath temperature of 80 °C and then for 4 hours at a bath temperature of 100 °C. When cold, the glacial acetic acid is removed by distillation under a waterpump vacuum, the solid residue is taken up in ethyl acetate, and the mixture is washed in succession with 10% sodium bicarbonate solution and saturated sodium chloride solution. The organic phase is dried over sodium sulfate. The mixture is concentrated in vacuo, and the residue is recrystallised from diisopropyl ether. 6 g of the title compound of m.p. 147-148 °C are obtained.

Example H6: Preparation of oxetan-3'-yl 2-chloro-4-fluoro-5-(1,2-cyclohex-1-enedicarboximido)benzoate (Compound No. 2.004):

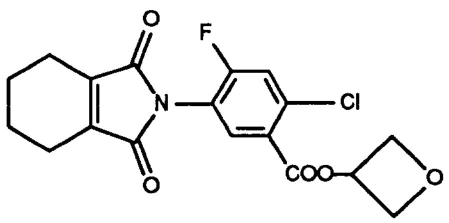

(2.004)

A solution of 6.8 g of 2-chloro-4-fluoro-5-(1,2-cyclohex-1-enedicarboximido)benzoyl chloride in 50 ml of toluene is added dropwise with stirring at a temperature of +20 °C to a solution of 1.8 g of 3-oxetanol, 2.8 ml of triethylamine and 50 ml of n-hexane. The mixture is subsequently filtered at room temperature, and the filtrate is washed with water. After evaporation followed by drying over sodium sulfate, the crude product obtained is purified by means of silica gel chromatography (ethyl acetate/n-hexane 1:3). 4.1 g of oxetan-3'-yl 2-chloro-4-fluoro-5-(1,2-cyclohex-1-enedicarboxim ido)benzoate (Compound No. 2.004) is obtained.

The compounds of Tables 1 to 5 can be prepared analogously to the above examples and the preparation processes mentioned in the description:

Table 1:

Compounds of the formula I

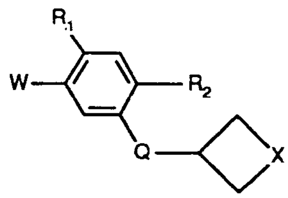 (I):

| Comp. No. | W | $R_1$ | $R_2$ | Q | X | Physical data |
|---|---|---|---|---|---|---|
| 1.001 | 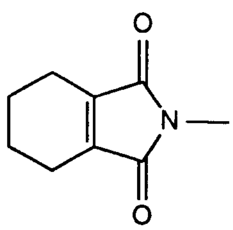 | H | Cl | O | O | |
| 1.002 | 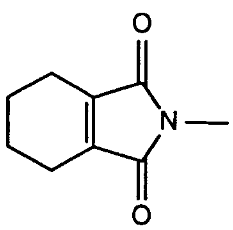 | H | Cl | O | S | |
| 1.003 | 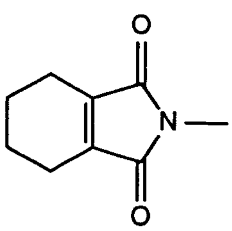 | H | Cl | S | O | |

| Comp. No. | W | $R_1$ | $R_2$ | Q | X | Phys. Data |
|---|---|---|---|---|---|---|
| 1.004 | 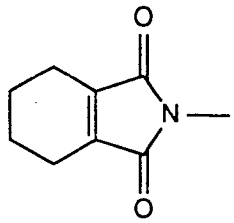 | H | Cl | S | S | |
| 1.005 | 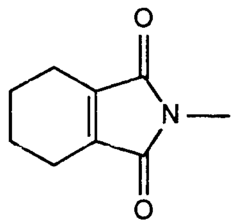 | F | Cl | O | O | m.p. 147-148°C |
| 1.006 | 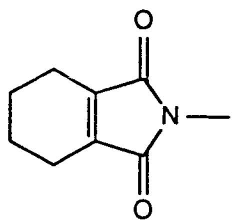 | F | Cl | O | S | |
| 1.007 | 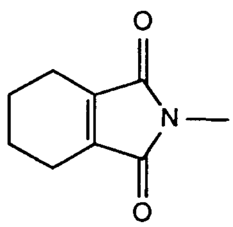 | F | Cl | S | O | |
| 1.008 | 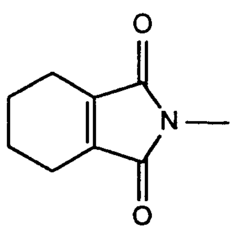 | F | Cl | S | S | |
| 1.009 | 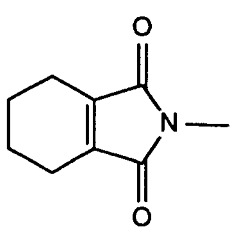 | F | Br | O | O | |

| Comp. No. | W | $R_1$ | $R_2$ | Q | X | Phys. Data |
|---|---|---|---|---|---|---|
| 1.010 | 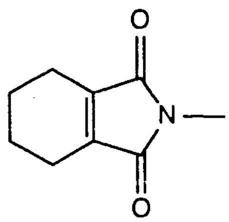 | F | CN | O | O | |
| 1.011 | 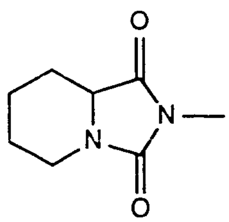 | F | Cl | O | O | |
| 1.012 | 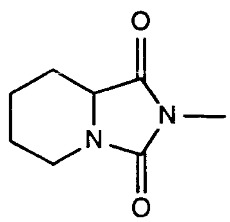 | F | Cl | O | S | |
| 1.013 | 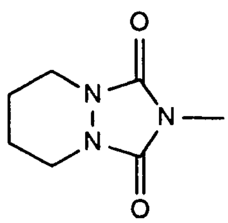 | F | Cl | O | O | |
| 1.014 | 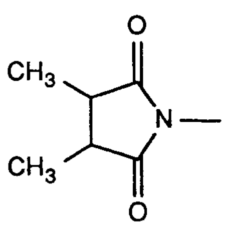 | F | Cl | O | O | |
| 1.015 | 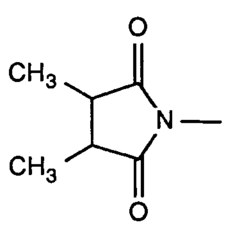 | F | Cl | O | S | |

| Comp. No. | W | $R_1$ | $R_2$ | Q | X | Phys. Data |
|---|---|---|---|---|---|---|
| 1.016 | 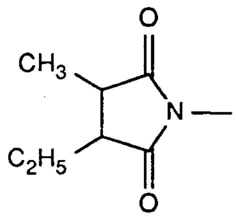 | F | Cl | O | O | |
| 1.017 | 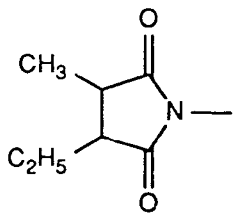 | F | Cl | O | S | |
| 1.018 | 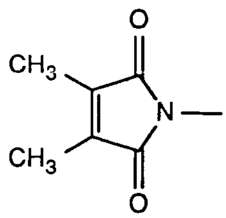 | H | Cl | O | O | |
| 1.019 | 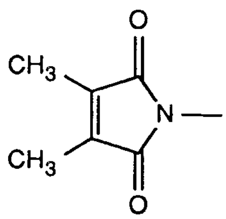 | H | Cl | O | S | |
| 1.020 | 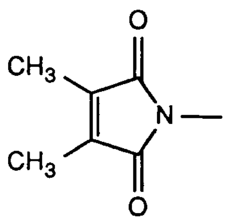 | F | Cl | O | O | |
| 1.021 | 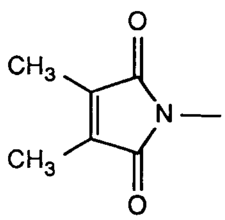 | F | Cl | O | S | |

| Comp. No. | W | $R_1$ | $R_2$ | Q | X | Phys. Data |
|---|---|---|---|---|---|---|
| 1.022 | 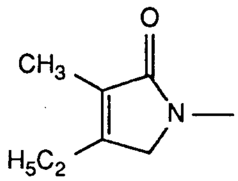 | H | Cl | O | O | |
| 1.023 | 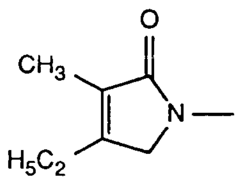 | H | Cl | O | S | |
| 1.024 | 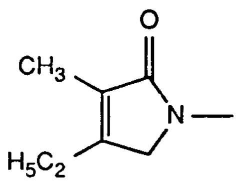 | F | Cl | O | O | |
| 1.025 | 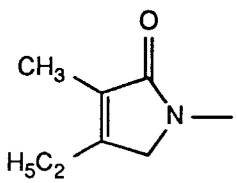 | F | Cl | O | S | |
| 1.026 | 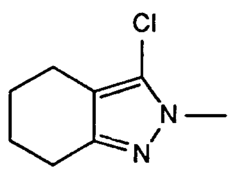 | F | Cl | O | O | |
| 1.027 | 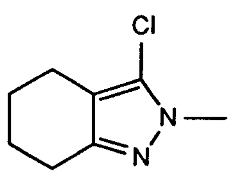 | F | Cl | O | S | |
| 1.028 | 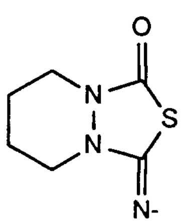 | H | Cl | O | O | |

| Comp. No. | W | $R_1$ | $R_2$ | Q | X | Phys. Data |
|---|---|---|---|---|---|---|
| 1.029 | 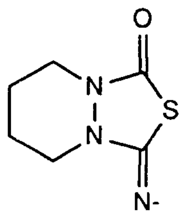 | H | Cl | O | S | |
| 1.030 | 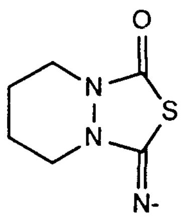 | H | Cl | S | O | |
| 1.031 | 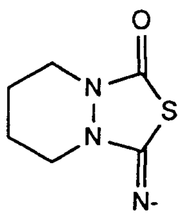 | H | Cl | S | S | |
| 1.032 | 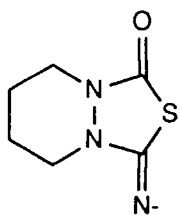 | F | Cl | O | O | |
| 1.033 | 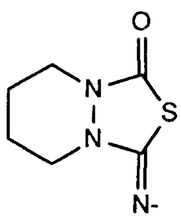 | F | Cl | O | S | |
| 1.034 | 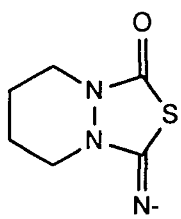 | F | Cl | S | O | |

| Comp. No. | W | $R_1$ | $R_2$ | Q | X | Phys. Data |
|---|---|---|---|---|---|---|
| 1.035 | 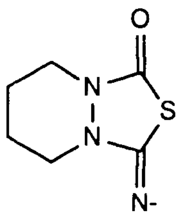 | F | Cl | S | S | |
| 1.036 | 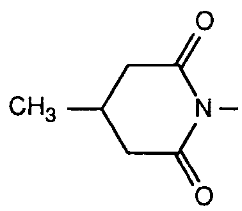 | F | Cl | O | O | |
| 1.037 | 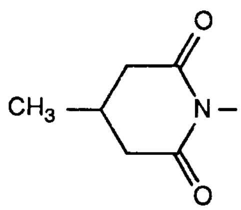 | F | Cl | O | S | |
| 1.038 | 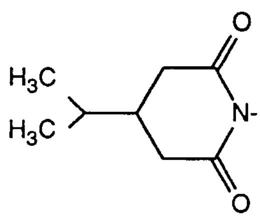 | F | Cl | O | O | |
| 1.039 | 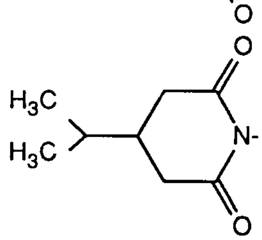 | F | Cl | O | S | |
| 1.040 | 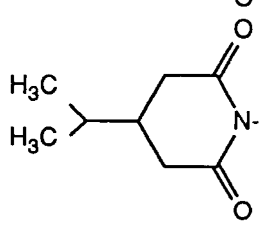 | F | Cl | S | O | |
| 1.041 | 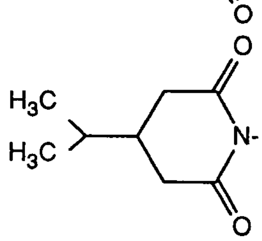 | F | Cl | S | S | |

| Comp. No. | W | $R_1$ | $R_2$ | Q | X | Phys. Data |
|---|---|---|---|---|---|---|
| 1.042 | 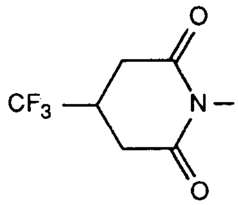 | H | Cl | O | O | |
| 1.043 | 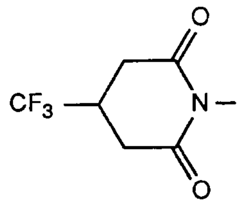 | H | Cl | O | S | |
| 1.044 | 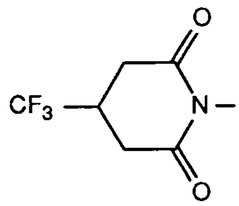 | H | Cl | S | O | |
| 1.045 | 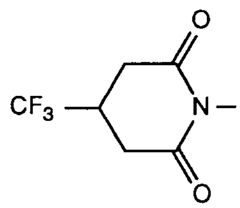 | H | Cl | S | S | |
| 1.046 | 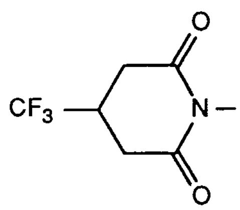 | F | Cl | O | O | m.p. 190-192°C |
| 1.047 | 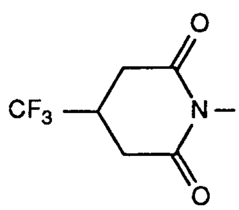 | F | Cl | O | S | |

| | | | | | |
|---|---|---|---|---|---|
| 1.048 | 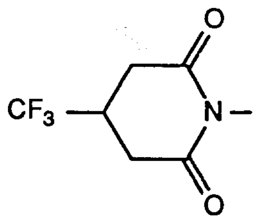 | F | Cl | S | O |
| 1.049 | 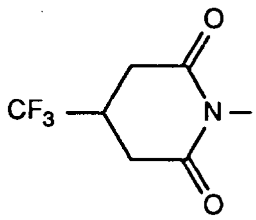 | F | Cl | S | S |

Table 2: Compounds of the formula Ia

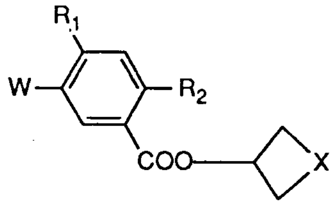

(Ia)

| Comp. No. | W | $R_1$ | $R_2$ | X | Physical data |
|---|---|---|---|---|---|
| 2.001 | 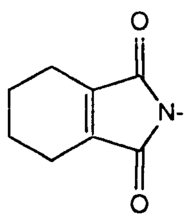 | H | Cl | O | |
| 2.002 | 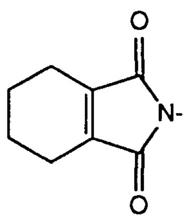 | H | Cl | S | |
| 2.003 | 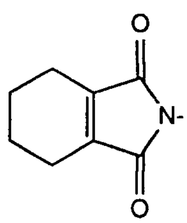 | H | Br | O | |
| 2.004 | 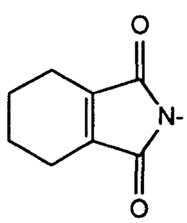 | F | Cl | O | m.p. 145°C |

| Comp. No. | W | $R_1$ | $R_2$ | X | Phys. Data |
|---|---|---|---|---|---|
| 2.005 | 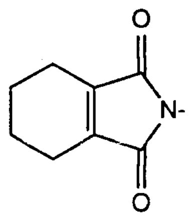 | F | Cl | S | m.p. 150-151°C |
| 2.006 | 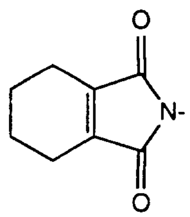 | F | Br | O | |
| 2.007 | 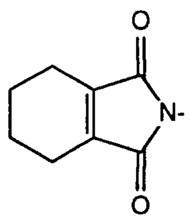 | F | Br | S | |
| 2.008 | 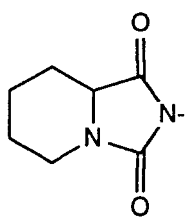 | F | Cl | O | |
| 2.009 | 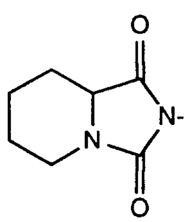 | F | Cl | S | |
| 2.010 | 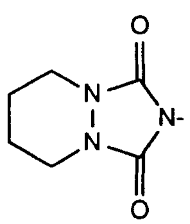 | F | Cl | O | |

| Comp. No. | W | $R_1$ | $R_2$ | X | Phys. Data |
|---|---|---|---|---|---|
| 2.011 | 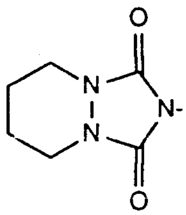 | F | Cl | S | |
| 2.012 | 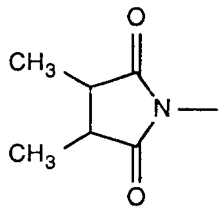 | F | Cl | O | |
| 2.013 | 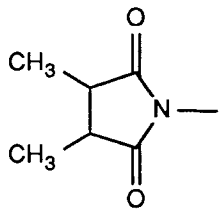 | F | Cl | S | |
| 2.014 | 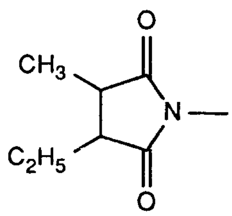 | H | Cl | O | |
| 2.015 | 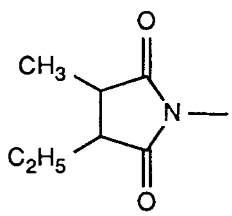 | H | Cl | S | |
| 2.016 | 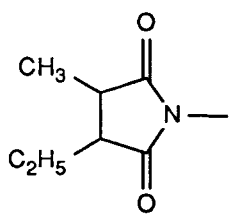 | F | Cl | O | |

| Comp. No. | W | $R_1$ | $R_2$ | X | Phys. Data |
|---|---|---|---|---|---|
| 2.017 | 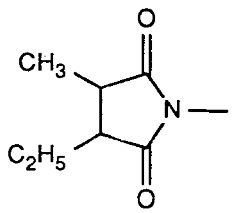 | F | Cl | S | |
| 2.018 | 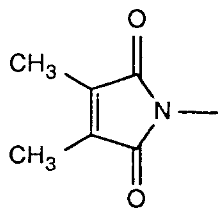 | H | Cl | O | |
| 2.019 | 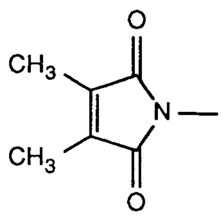 | H | Cl | S | |
| 2.020 | 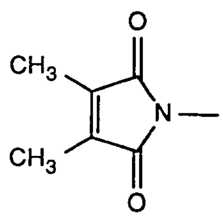 | F | Cl | O | |
| 2.021 | 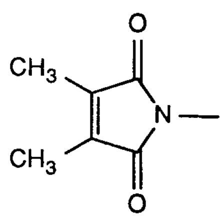 | F | Cl | S | |
| 2.022 | 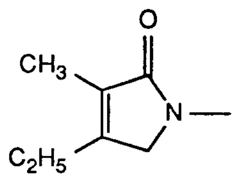 | H | Cl | O | |

| Comp. No. | W | $R_1$ | $R_2$ | X | Phys. Data |
|---|---|---|---|---|---|
| 2.023 | 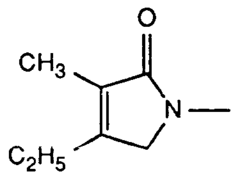 | H | Cl | S | |
| 2.024 | 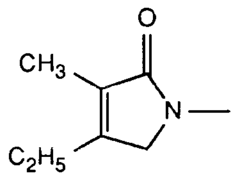 | F | Cl | O | |
| 2.025 | 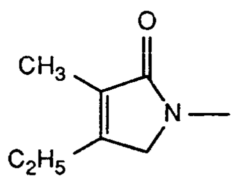 | F | Cl | S | |
| 2.026 | 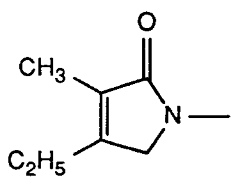 | F | Br | O | |
| 2.027 | 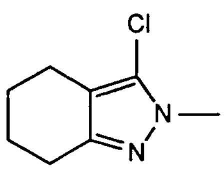 | F | Cl | O | |
| 2.028 | 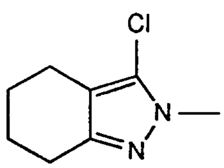 | F | Cl | S | |
| 2.029 | 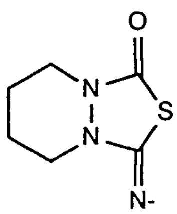 | H | Cl | O | |

| Comp. No. | W | $R_1$ | $R_2$ | X | Phys. Data |
|---|---|---|---|---|---|
| 2.030 | 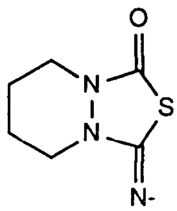 | H | Cl | S | |
| 2.031 | 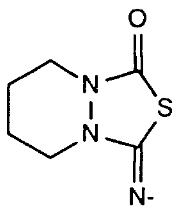 | F | Cl | O | m.p. 133-134°C |
| 2.032 | 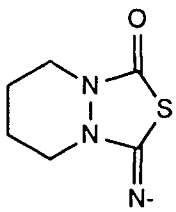 | F | Cl | S | |
| 2.033 | 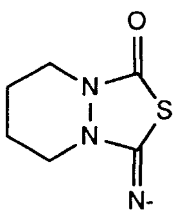 | F | Br | O | |
| 2.034 | 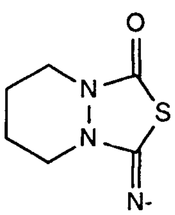 | F | Br | S | |
| 2.035 | 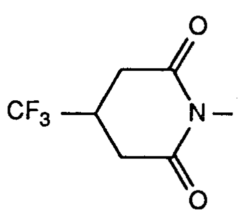 | H | Cl | O | |

| Comp. No. | W | $R_1$ | $R_2$ | X | Phys. Data |
|---|---|---|---|---|---|
| 2.036 | 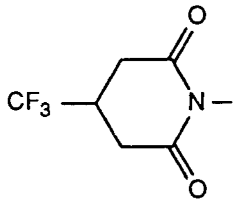 | H | Cl | S | |
| 2.037 | 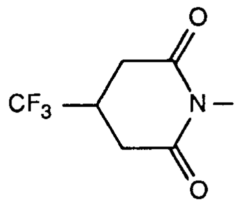 | F | Cl | O | m.p. 143-146°C |
| 2.038 | 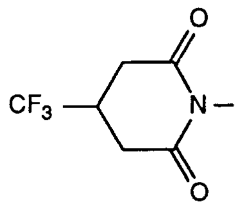 | F | Cl | S | m.p. 159-160°C |
| 2.039 | 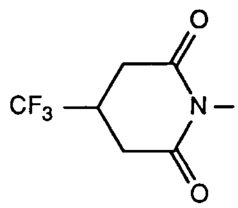 | F | Br | O | |
| 2.040 | 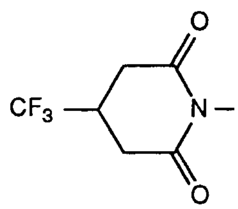 | F | Br | S | |
| 2.041 | 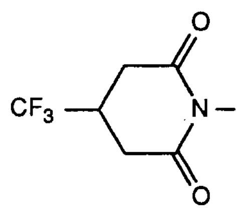 | F | CN | O | |

Table 3: Compounds of the formula Ib:

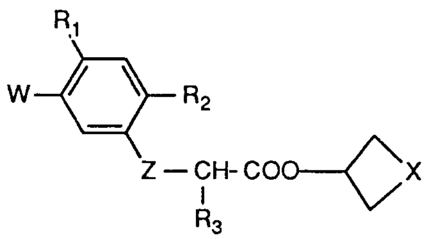

(Ib)

| Comp. No. | W | $R_1$ | $R_2$ | $R_3$ | Z | X | Physical Data |
|---|---|---|---|---|---|---|---|
| 3.001 | 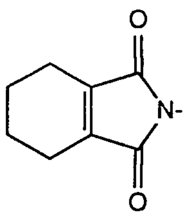 | H | Cl | H | O | O | |
| 3.002 | 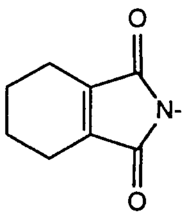 | H | Cl | H | O | S | |
| 3.003 | 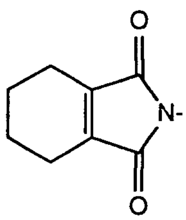 | H | Cl | H | S | O | |
| 3.004 | 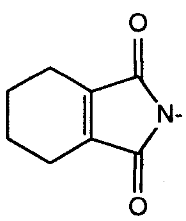 | H | Cl | H | S | S | |

| Comp. No. | W | $R_1$ | $R_2$ | $R_3$ | Z | X | Phys. Data |
|---|---|---|---|---|---|---|---|
| 3.005 | 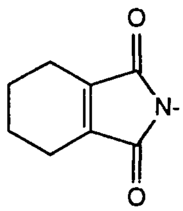 | H | Br | H | O | O | |
| 3.006 | 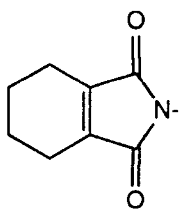 | H | CN | H | O | O | |
| 3.007 | 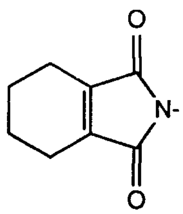 | H | Cl | $CH_3$ | O | O | |
| 3.008 | 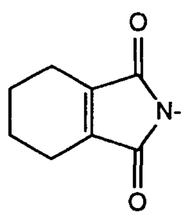 | H | Cl | $CH_3$ | O | S | |
| 3.009 | 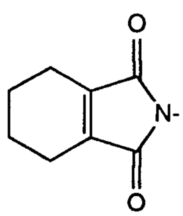 | H | Cl | $CH_3$ | S | O | |
| 3.010 | 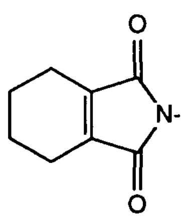 | H | Cl | $CH_3$ | S | S | |

| Comp. No. | W | $R_1$ | $R_2$ | $R_3$ | Z | X | Phys. Data |
|---|---|---|---|---|---|---|---|
| 3.011 | 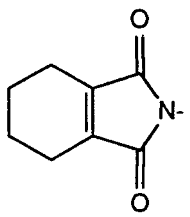 | H | CN | H | O | O | |
| 3.012 | 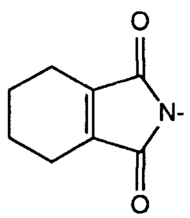 | F | Cl | H | O | O | |
| 3.013 | 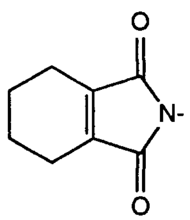 | F | Cl | H | O | S | |
| 3.014 | 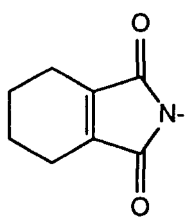 | F | Cl | H | S | O | |
| 3.015 | 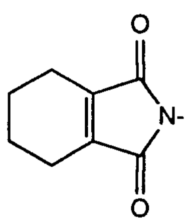 | F | Cl | H | S | S | |
| 3.016 | 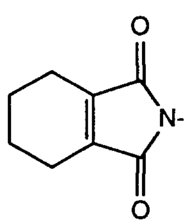 | F | Cl | $CH_3$ | O | O | |

| Comp. No. | W | $R_1$ | $R_2$ | $R_3$ | Z | X | Phys. Data |
|---|---|---|---|---|---|---|---|
| 3.017 | 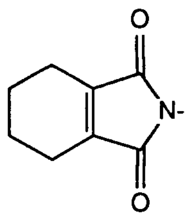 | F | Cl | $CH_3$ | O | S | |
| 3.018 | 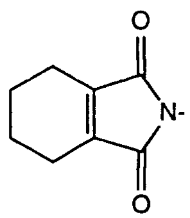 | F | Cl | $CH_3$ | S | O | |
| 3.019 | 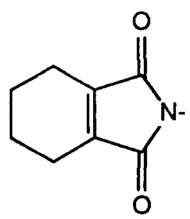 | F | Cl | $CH_3$ | S | S | |
| 3.020 | 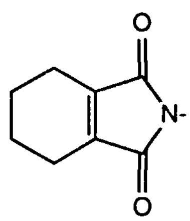 | F | Br | H | O | O | |
| 3.021 | 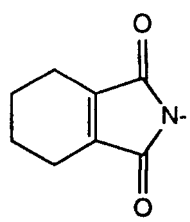 | F | Br | $CH_3$ | O | O | |
| 3.022 | 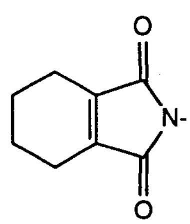 | F | CN | $CH_3$ | O | O | |

| Comp. No. | W | $R_1$ | $R_2$ | $R_3$ | Z | X | Phys. Data |
|---|---|---|---|---|---|---|---|
| 3.023 | 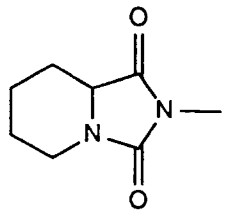 | F | Cl | H | O | O | |
| 3.024 | 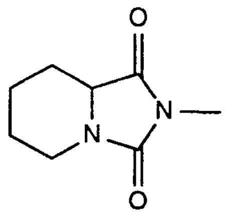 | F | Cl | $CH_3$ | O | O | |
| 3.025 | 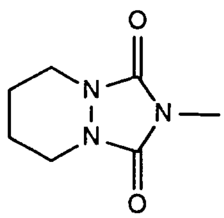 | F | Cl | H | O | O | |
| 3.026 | 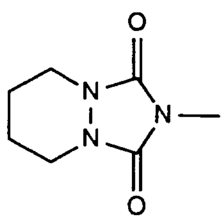 | F | Cl | $CH_3$ | O | O | |
| 3.027 | 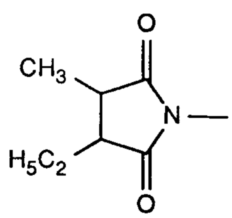 | F | Cl | H | O | O | |
| 3.028 | 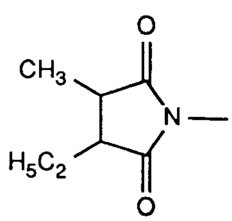 | F | Cl | $CH_3$ | O | O | |

| Comp. No. | W | $R_1$ | $R_2$ | $R_3$ | Z | X | Phys. Data |
|---|---|---|---|---|---|---|---|
| 3.029 | 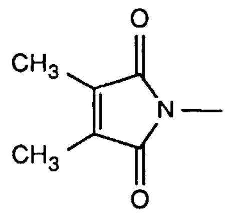 | F | Cl | H | O | O | |
| 3.030 | 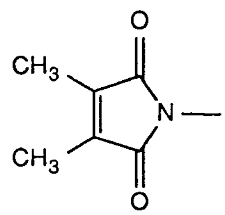 | F | Cl | $CH_3$ | O | O | |
| 3.031 | 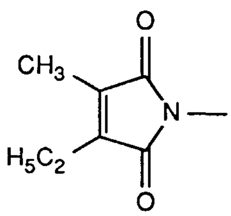 | F | Cl | H | O | O | |
| 3.032 | 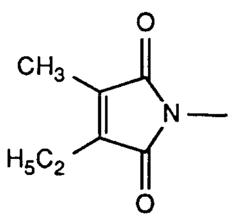 | F | Cl | $CH_3$ | O | O | |
| 3.033 | 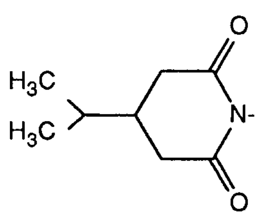 | F | Cl | H | O | O | |
| 3.034 | 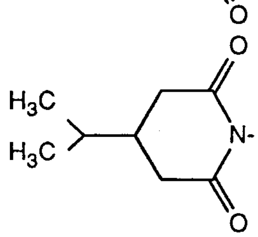 | F | Cl | $CH_3$ | O | O | |

| Comp. No. | W | $R_1$ | $R_2$ | $R_3$ | Z | X | Phys. Data |
|---|---|---|---|---|---|---|---|
| 3.035 | 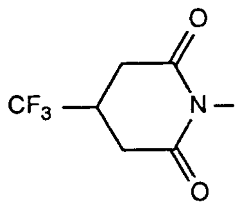 | H | Cl | H | O | O | |
| 3.036 | 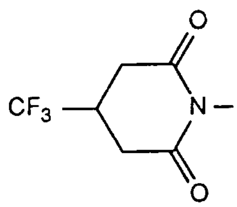 | H | Cl | H | O | S | |
| 3.037 | 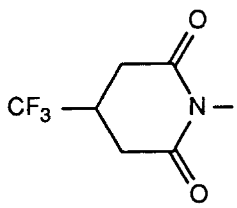 | H | Cl | $CH_3$ | O | O | |
| 3.038 | 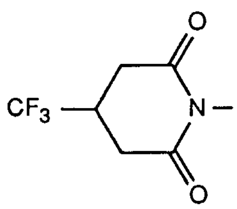 | H | Cl | $CH_3$ | O | S | |
| 3.039 | 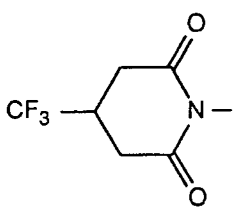 | F | Cl | H | O | O | |
| 3.040 | 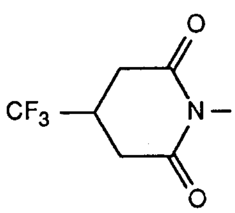 | F | Cl | H | O | S | |

| Comp. No. | W | $R_1$ | $R_2$ | $R_3$ | Z | X | Phys. Data |
|---|---|---|---|---|---|---|---|
| 3.041 | 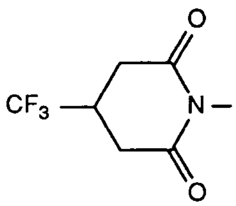 | F | Cl | $CH_3$ | O | O | |
| 3.042 | 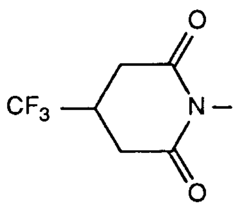 | F | Cl | $CH_3$ | O | S | |
| 3.043 | 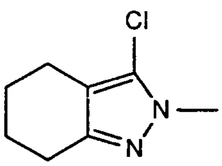 | F | Cl | H | O | O | |
| 3.044 | 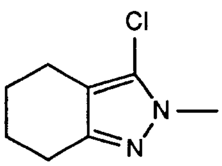 | F | Cl | $CH_3$ | O | O | |
| 3.045 | 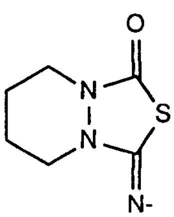 | H | Cl | H | O | O | |
| 3.046 | 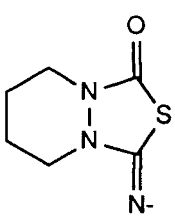 | H | Cl | H | O | S | |
| 3.047 | 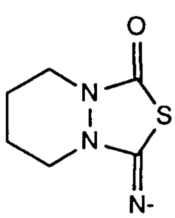 | H | Cl | $CH_3$ | O | O | |

| Comp. No. | W | $R_1$ | $R_2$ | $R_3$ | Z | X | Phys. Data |
|---|---|---|---|---|---|---|---|
| 3.048 | 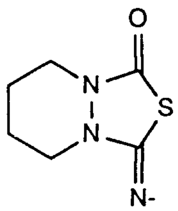 | H | Cl | $CH_3$ | O | S | |
| 3.049 | 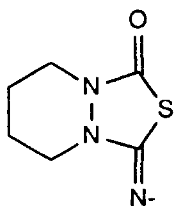 | F | Cl | H | O | O | |
| 3.050 | 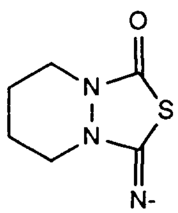 | F | Cl | H | O | S | |
| 3.051 | 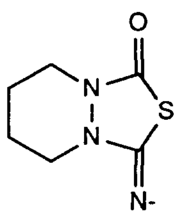 | F | Cl | $CH_3$ | O | O | |
| 3.052 | 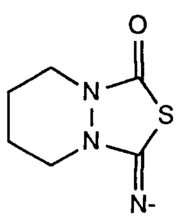 | F | Cl | $CH_3$ | O | S | |
| 3.053 | 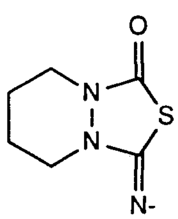 | F | Cl | H | S | O | |

| Comp. No. | W | $R_1$ | $R_2$ | $R_3$ | Z | X | Phys. Data |
|---|---|---|---|---|---|---|---|
| 3.054 | 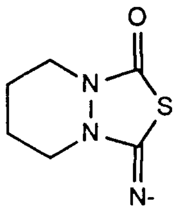 | F | CL | H | S | S | |
| 3.055 | 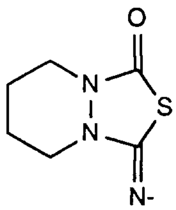 | F | CL | $CH_3$ | S | O | |
| 3.056 | 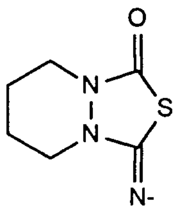 | F | Cl | $CH_3$ | S | S | |

Table 4: Compounds of the formula Ic:

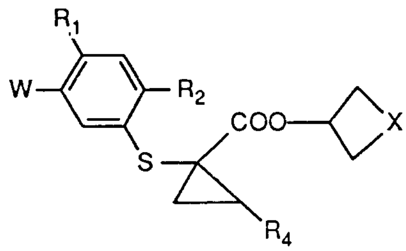

(Ic)

| Comp. No. | W | $R_1$ | $R_2$ | $R_4$ | X | Physical data |
|---|---|---|---|---|---|---|
| 4.001 | 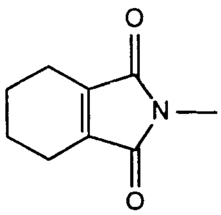 | H | Cl | H | O | |
| 4.002 | 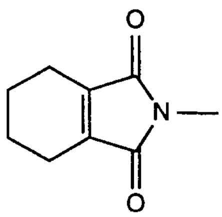 | H | Cl | H | S | |
| 4.003 | 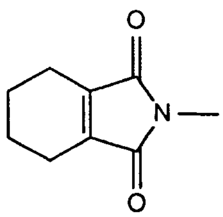 | H | Cl | F | O | |
| 4.004 | 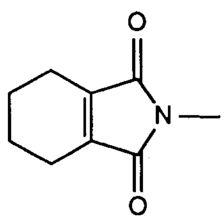 | H | Cl | F | S | |

| Comp. No. | W | $R_1$ | $R_2$ | $R_4$ | X | Phys. Data |
|---|---|---|---|---|---|---|
| 4.005 | 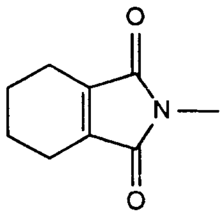 | F | Cl | H | O | |
| 4.006 | 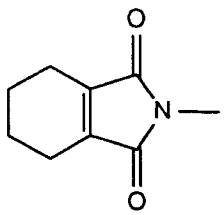 | F | Cl | H | S | |
| 4.007 | 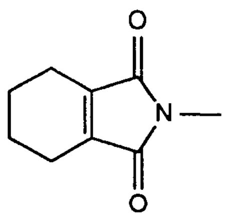 | F | Cl | F | O | |
| 4.008 | 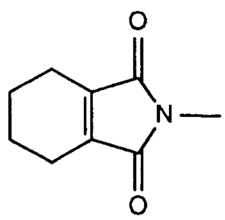 | F | Cl | F | S | |
| 4.009 | 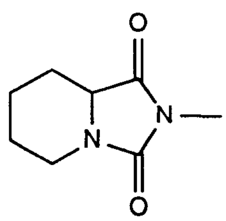 | F | Cl | H | O | |
| 4.010 | 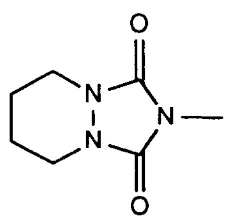 | F | Cl | H | O | |

| Comp. No. | W | $R_1$ | $R_2$ | $R_4$ | X | Phys. Data |
|---|---|---|---|---|---|---|
| 4.011 | 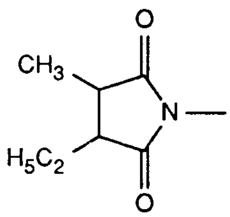 | F | Cl | H | O | |
| 4.012 | 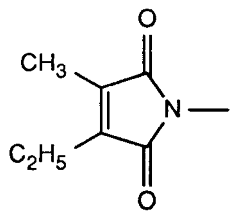 | F | Cl | H | O | |
| 4.013 | 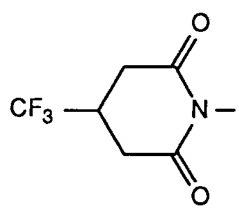 | F | Cl | H | O | |
| 4.014 | 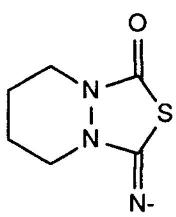 | F | Cl | H | O | m.p. 117-119°C |
| 4.015 | 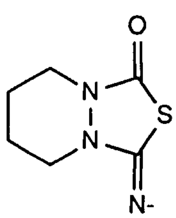 | F | Cl | H | S | |
| 4.016 | 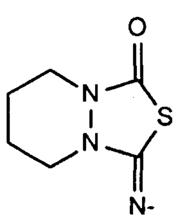 | F | Cl | F | O | |

Table 5: Compounds of the formula Id:

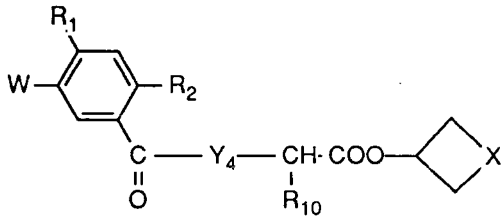

(Id)

| Comp. No. | W | $Y_4$ | $R_1$ | $R_2$ | $R_{10}$ | X | Physical data |
|---|---|---|---|---|---|---|---|
| 5.001 | 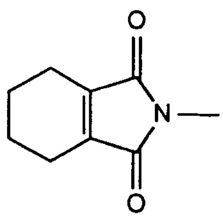 | O | F | Cl | H | O | |
| 5.002 | 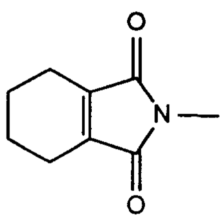 | S | F | Cl | H | O | |
| 5.003 | 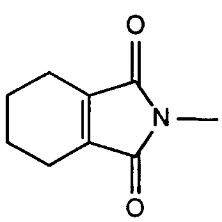 | O | F | Cl | $CH_3$ | O | |
| 5.004 | 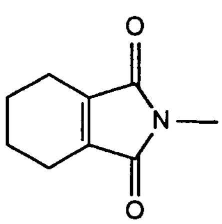 | S | F | Cl | $CH_3$ | O | |

| Comp. No. | W | $Y_4$ | $R_1$ | $R_2$ | $R_{10}$ | X | Phys. Data |
|---|---|---|---|---|---|---|---|
| 5.005 | 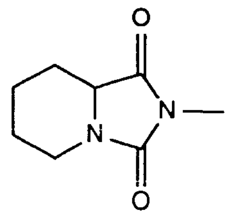 | O | F | Cl | H | O | |
| 5.006 | 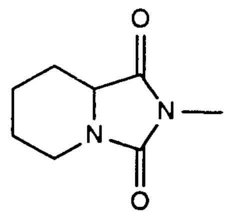 | S | F | Cl | H | O | |
| 5.007 | 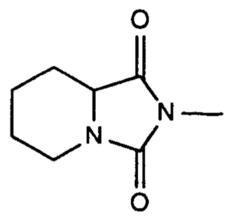 | O | F | Cl | $CH_3$ | O | |
| 5.008 | 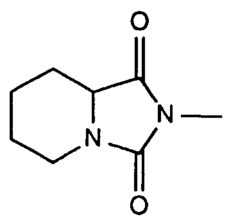 | S | F | Cl | $CH_3$ | O | |
| 5.009 | 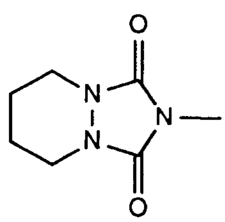 | O | F | Cl | H | O | |
| 5.010 | 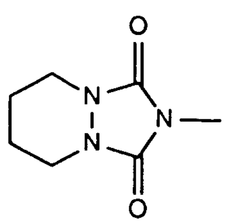 | S | F | Cl | H | O | |

| Comp. No. | W | $Y_4$ | $R_1$ | $R_2$ | $R_{10}$ | X | Phys. Data |
|---|---|---|---|---|---|---|---|
| 5.011 | 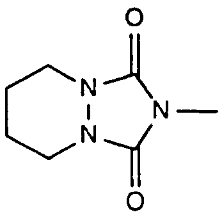 | O | F | Cl | $CH_3$ | O | |
| 5.012 | 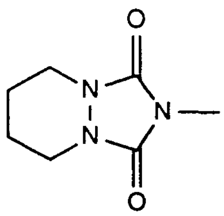 | S | F | Cl | $CH_3$ | O | |
| 5.013 | 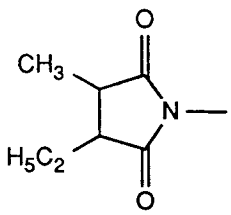 | O | F | Cl | H | O | |
| 5.014 | 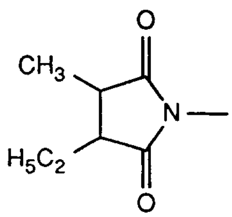 | S | F | Cl | H | O | |
| 5.015 | 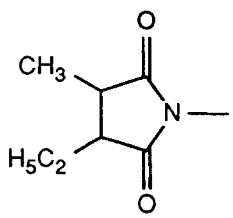 | O | F | Cl | $CH_3$ | O | |
| 5.016 | 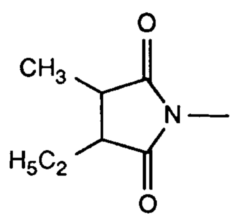 | S | F | Cl | $CH_3$ | O | |

| Comp. No. | W | $Y_4$ | $R_1$ | $R_2$ | $R_{10}$ | X | Phys. Data |
|---|---|---|---|---|---|---|---|
| 5.017 | 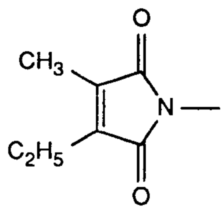 | O | F | Cl | H | O | |
| 5.018 | 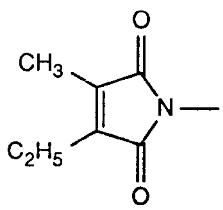 | S | F | Cl | H | O | |
| 5.019 | 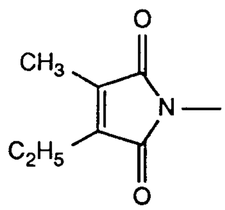 | O | F | Cl | $CH_3$ | O | |
| 5.020 | 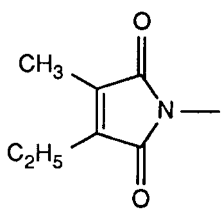 | S | F | Cl | $CH_3$ | O | |
| 5.021 | 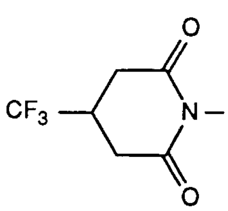 | O | F | Cl | H | O | |
| 5.022 | 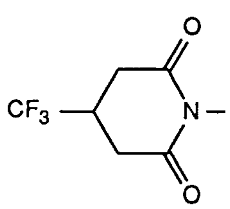 | S | F | Cl | H | O | |

| Comp. No. | W | $Y_4$ | $R_1$ | $R_2$ | $R_{10}$ | X | Phys. Data |
|---|---|---|---|---|---|---|---|
| 5.023 | 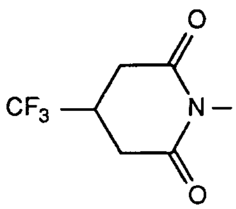 | O | F | Cl | $CH_3$ | O | |
| 5.024 | 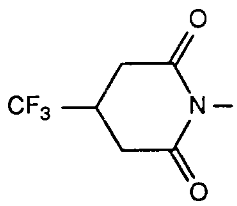 | S | F | Cl | $CH_3$ | O | |
| 5.025 | 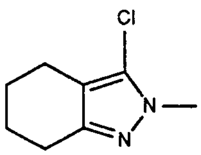 | O | F | Cl | H | O | |
| 5.026 | 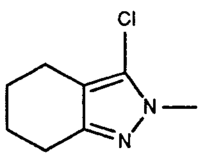 | S | F | Cl | H | O | |
| 5.027 | 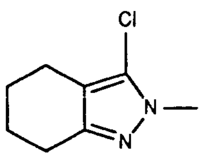 | O | F | Cl | $CH_3$ | O | |
| 5.028 | 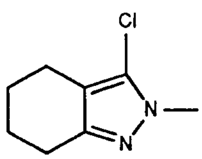 | S | F | Cl | $CH_3$ | O | |
| 5.029 | 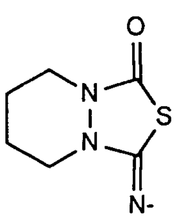 | O | F | Cl | H | O | |

| Comp. No. | W | $Y_4$ | $R_1$ | $R_2$ | $R_{10}$ | X | Phys. Data |
|---|---|---|---|---|---|---|---|
| 5.030 | 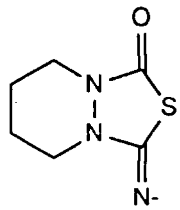 | S | F | Cl | H | O | |
| 5.031 | 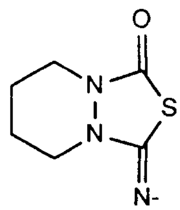 | O | F | Cl | $CH_3$ | O | |
| 5.032 | 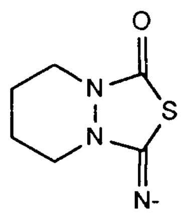 | S | F | Cl | $CH_3$ | O | |
| 5.033 | 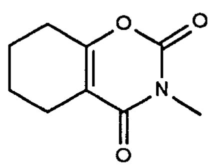 | O | F | Cl | H | O | |
| 5.034 | 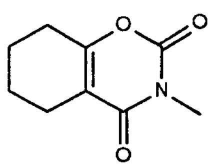 | S | F | Cl | H | O | |
| 5.035 | 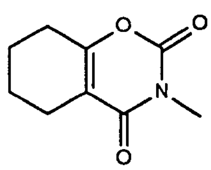 | O | F | Cl | $CH_3$ | O | |

| Comp. No. | W | $Y_4$ | $R_1$ | $R_2$ | $R_{10}$ | X | Phys. Data |
|---|---|---|---|---|---|---|---|
| 5.036 | 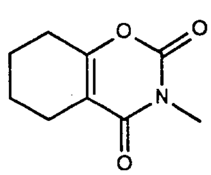 | S | F | Cl | $CH_3$ | O | |

Formulation examples of liquid active ingredients of the formula I (% = percent by weight)

| 1. Emulsion concentrates | a) | b) | c) |
|---|---|---|---|
| Active ingredient of Table 1-5 | 25% | 40% | 50% |
| Calcium dodecylbenzenesulfonate | 5% | 8% | 6% |
| Castor oil polyethylene glycol ether (36 mol of EO) | 5% | - | - |
| Tributylphenol polyethylene glycol ether (30 mol of EO) | - | 12% | 4% |
| Cyclohexanone | - | 15% | 20% |
| Xylene mixture | 65% | 25% | 20% |

Emulsions of any desired concentration can be prepared from such concentrates by dilution with water.

| 2. Solutions | a) | b) | c) | d) |
|---|---|---|---|---|
| Active ingredient of Table 1-5 | 80% | 10% | 5% | 95% |
| Propylene glycol monomethyl ether | 20% | - | - | - |
| Polyethylene glycol MW 400 | - | 70% | - | - |
| N-Methyl-2-pyrrolidone | - | 20% | - | - |
| Epoxidised coconut oil | - | - | 1% | 5% |
| Petroleum spirit (boiling range 160-190 ° C) | - | - | 94% | - |

The solutions are suitable for use in the form of microdrops.

| 3. Granules | a) | b) | c) | d) |
|---|---|---|---|---|
| Active ingredient of Table 1-5 | 5% | 10% | 8% | 21% |
| Kaolin | 94% | - | 79% | 54% |
| Highly-disperse silica | 1% | - | 13% | 7% |
| Attapulgite | - | 90% | - | 18% |

The active ingredient is dissolved in methylene chloride, the solution is sprayed onto the carrier, and the solvent is subsequently removed by evaporation in vacuo.

| 4. Dusts | a) | b) |
|---|---|---|
| Active ingredient of Table 1-5 | 2% | 5% |
| Highly-disperse silica | 1% | 5% |
| Talc | 97% | - |
| Kaolin | - | 90% |

Ready-for-use dusts are obtained by intimately mixing the carriers with the active ingredient.

Formulation examples of solid active ingredients of the formula I (% = percent by weight)

| 5. Wettable powders | a) | b) | c) |
|---|---|---|---|
| Active ingredient of Table 1-5 | 25% | 50% | 75% |
| Sodium ligninsulfonate | 5% | 5% | - |
| Sodium lauryl sulfate | 3% | - | 5% |
| Sodium diisobutylnaphthalene-sulfonate | - | 6% | 10% |
| Octylphenol polyethylene glycol ether (7-8 mol of EO) | - | 2% | - |
| Highly-disperse silica | 5% | 10% | 10% |
| Kaolin | 62% | 27% | - |

The active ingredient is mixed thoroughly with the additives, and the mixture is ground thoroughly in a suitable mill. This gives wettable powders which can be diluted with water to give suspensions of any desired concentration.

| 6. Emulsion concentrate | |
|---|---|
| Active ingredient of Table 1-5 | 10% |
| Octylphenol polyethylene glycol ether (4-5 mol of EO) | 3% |
| Calcium dodecylbenzenesulfonate | 3% |
| Castor oil polyglycol ether (36 mol of EO) | 4% |
| Cyclohexanone | 30% |
| Xylene mixture | 50% |

Emulsions of any desired concentration can be prepared from this concentrate by dilution with water.

| 7. Dusts | a) | b) |
|---|---|---|
| Active ingredient of Table 1-5 | 5% | 8% |
| Talc | 95% | - |
| Kaolin | - | 92% |

Ready-for-use dusts are obtained by mixing the active ingredient with the carriers and grinding the mixture on a suitable mill.

| 8. Extruder granules | |
|---|---|
| Active ingredient of Table 1-5 | 10% |
| Sodium ligninsulfonate | 2% |
| Carboxymethylcellulose | 1% |
| Kaolin | 87% |

The active ingredient is mixed with the additives, and the mixture is ground and moistened with water. This mixture is extruded and subsequently dried in a stream of air.

| 9. Coated granules | |
|---|---|
| Active ingredient of Table 1-5 | 3% |
| Polyethylene glycol (MW 200) | 3% |
| Kaolin | 94% |

In a mixer, the kaolin which is moistened with polyethylene glycol is coated uniformly with the finely-ground active ingredient. In this manner, dust-free coated granules are obtained.

| 10. Suspension concentrate | |
|---|---|
| Active ingredient of Table 1-5 | 40% |
| Propylene glycol | 10% |
| Nonylphenol polyethylene glycol ether (15 mol of EO) | 6% |
| Sodium ligninsulfonate | 10% |
| Carboxymethylcellulose | 1% |
| Silicone oil in the form of a 75% aqueous emulsion | 1% |
| Water | 32% |

The finely-ground active ingredient is mixed intimately with the additives. This gives a suspension concentrate from which suspensions of any desired concentration can be prepared by dilution with water. The compounds of the formula I are employed as pure active ingredients or, preferably, as a composition together with the auxiliaries customary in the art of formulation, and are therefore processed in a known manner to give, for example, emulsion concentrates, directly sprayable or dilutable solutions, dilute emulsions, wettable powders, soluble powders, dusts, granules and also encapsulations, for example in polymeric substances. The application methods such as spraying, atomising, dusting, scattering or pouring as well as the type of composition are selected to suit the intended aims and prevailing circumstances.

## Biological Examples

### Example B1: Herbicidal action, pre-emergence

In a greenhouse, the test plants are sown into seed dishes, and immediately afterwards the soil surface is treated with an aqueous spray mixture at an application rate equivalent to 2 kg of active ingredient/hectare. The seed dishes are kept in the greenhouse at 22-25°C and 50-70% relative atmospheric humidity.

After 3 weeks, the herbicidal action is assessed using a nine-step rating scheme (1 = complete damage, 9 = no action) by comparison with an untreated control group.

Ratings of 1 to 4 (in particular 1 to 3) suggest a good to very good herbicidal action. Ratings from 6 to 9 (in particular 7 to 9) suggest a good tolerance (in particular in the case of crop plants).

In this test, the compounds of Tables 1-5 show a powerful herbicidal action. Individual results are compiled in Table B1:

Table B1: Herbicidal action, pre-emergence:

| Test plant: Active ingredient no. | Abutilon | Amaranthus | Chenopodium | Solanum | Chrysant. |
|---|---|---|---|---|---|
| 2.004 | 1 | 1 | 1 | 1 | 1 |
| 2.031 | 1 | 1 | 1 | 1 | 1 |

### Example B2: Herbicidal action, post-emergence (contact herbicide)

After emergence, a number of monocotyledon and dicotyledon weeds (in the 4-to 6-leaf stage) were sprayed with an aqueous dispersion of active ingredient at a dosage rate of 2 kg of active ingredient per hectare, and the plants were kept at 24°-26°C and 45-60% relative atmospheric humidity. 15 days after the treatment, the herbidical action is assessed using a nine-step rating scheme (1 = complete damage, 9 = no action) by comparison with an untreated control group.

Ratings of 1 to 4 (in particular 1 to 3) suggest a good to very good herbicidal action. Ratings from 6 to 9 (in particular 7 to 9) suggest a good tolerance (in particular in the case of crop plants).

In this test, the compounds of Tables 1-5 show a powerful herbicidal action. Individual results are compiled in Table B2:

Table B2: Herbicidal action, post-emergence:

| Test plant: Active ingredient No. | Avena | Setaria | Lolium | Solanum | Sinapis | Stellaria |
|---|---|---|---|---|---|---|
| 2.004 | 1 | 1 | 1 | 1 | 1 | 1 |
| 2.031 | 1 | 1 | 2 | 1 | 1 | 2 |

Example B3: Herbicidal action in paddy rice: The aquatic weeds Echinochloa crus galli and Monocharia vag. are sown in plastic beakers (surface area 60 $cm^2$, volume 500 ml). After sowing, the beakers are filled with water up to the soil surface. 3 days after sowing, the soil surface is flooded slightly (3-5 mm). Application is effected 3 days after sowing by spraying the test substances onto the containers. The dosage used corresponds to an amount of active ingredient of 2 kg a.i. per hectare. The beakers with the plants are then placed in the greenhouse under ideal growth conditions for the rice weeds, i.e. at 25°-30°C and high atmospheric humidity.

The tests are evaluated 3 weeks after application. The compounds of Table 1 damage the weeds.

**Claims**

**1.** An oxetane or thiaetane derivative of the formula I

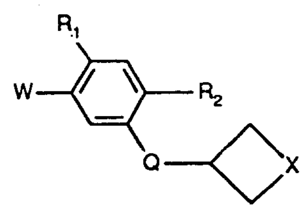

(I),

in which

Q is oxygen, sulfur, -COO-, -Z-CH($R_3$)-COO-,

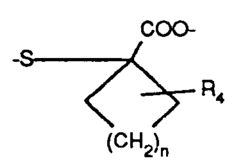

,

or CO-$Y_4$-CH($R_{10}$)-COO-;

W is

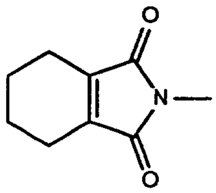 (W1), 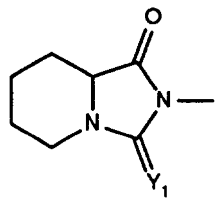 (W2), 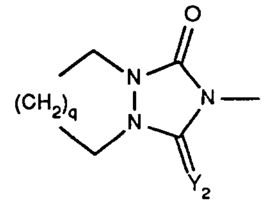 (W3),

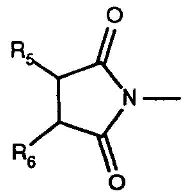 (W4), 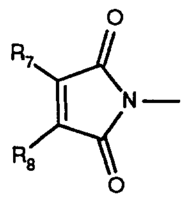 (W5), 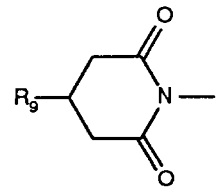 (W6),

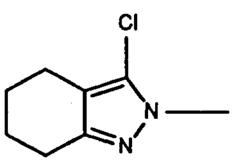 (W7), 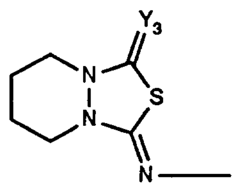 (W8) or 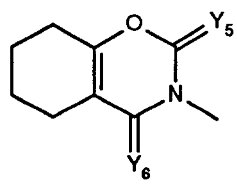 (W9) ; and

$R_1$ is hydrogen or fluorine; $R_2$ is halogen or cyano; $R_3$ and $R_{10}$ independently of one another are hydrogen or $C_1$-$C_3$alkyl; $R_4$ is hydrogen, fluorine, chlorine, bromine, $C_1$-$C_4$alkyl or trifluoromethyl; $R_5$ and $R_7$ independently of one another are $C_1$-$C_4$alkyl; $R_6$ and $R_8$ independently of one another are hydrogen or $C_1$-$C_4$alkyl; $R_9$ is hydrogen, $C_1$-$C_6$alkyl, $C_3$-$C_7$cycloalkyl, $C_1$-$C_4$alkoxy-$C_1$-$C_4$alkyl or $C_1$-$C_7$haloalkyl; X is oxygen or sulfur; $Y_1$, $Y_2$, $Y_3$, $Y_4$, $Y_5$ and $Y_6$ independently of one another are oxygen or sulfur; Z is oxygen or sulfur; n is 0; 1, 2, 3 or 4; and q is 1 or 2.

**2.** A compound according to claim 1, wherein $R_1$ is fluorine.

**3.** A compound according to claim 1, wherein $R_2$ is chlorine.

**4.** A compound according to claim 1, wherein W is $W_1$, $W_6$ or $W_8$.

**5.** A compound according to claim 4, wherein $R_9$ is trifluoromethyl.

**6.** A compound according to claim 1, wherein $Y_1$, $Y_2$ and $Y_3$ are oxygen.

**7.** A compound according to claim 1, wherein Q is the group

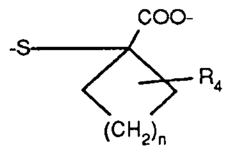

and $R_4$ is hydrogen and n is 0.

**8.** A compound according to claim 1, wherein Q is -COO- and X is oxygen.

**9.** A compound according to claim 8, wherein $R_1$ is fluorine and $R_2$ is chlorine.

**10.** A compound according to claim 8, wherein W is $W_8$ and $Y_3$ is oxygen.

**11.** A compound according to claim 1, wherein Q is the group -Z-CH($R_3$)-COO-.

**12.** A compound according to claim 1, wherein Q is the group CO-$Y_4$-CH($R_{10}$)-COO-.

**13.** 9-(4-Chloro-2-fluoro-5-oxetanyloxycarbonylphenylimino)-8-thia-1,6-diazabicyclo[4.3.0]nonan-7-one according to claim 1.

**14.** N-[4'-chloro-2'-fluoro-5'(3''-oxetanyl)oxyphenyl]-3,4,5,6-tetrahydrophthalimide according to claim 1.

**15.** A process for the preparation of the compounds of the formula I

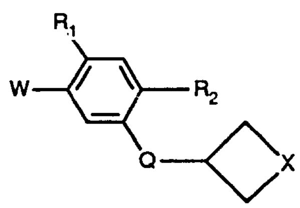

(I),

in which

Q is oxygen, sulfur, -COO-, -Z-CH($R_3$)-COO-,

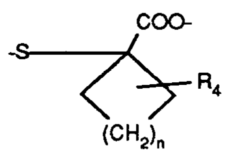

,

or CO-$Y_4$-CH($R_{10}$)-COO-;

W is

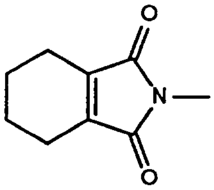 (W1), 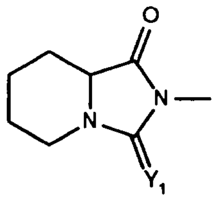 (W2), 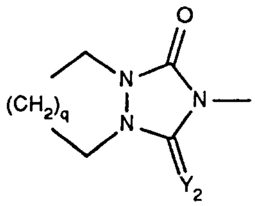 (W3),

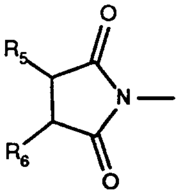 (W4), 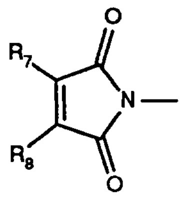 (W5), 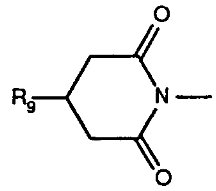 (W6),

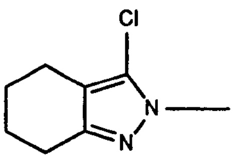 (W7), 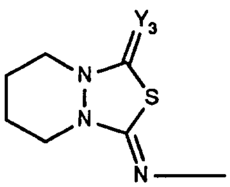 (W8) or 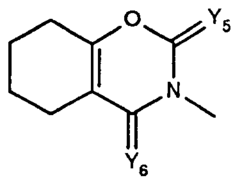 (W9) ;

and $R_1$ is hydrogen or fluorine; $R_2$ is halogen or cyano; $R_3$ and $R_{10}$ independently of one another are hydrogen or $C_1$-$C_3$alkyl; $R_4$ is hydrogen, fluorine, chlorine, bromine, $C_1$-$C_4$alkyl or trifluoromethyl; $R_5$ and $R_7$ independently of one another are $C_1$-$C_4$alkyl; $R_6$ and $R_8$ independently of one another are hydrogen or $C_1$-$C_4$alkyl; $R_9$ is hydrogen, $C_1$-$C_6$alkyl, $C_3$-$C_7$cycloalkyl, $C_1$-$C_4$alkoxy-$C_1$-$C_4$alkyl or $C_1$-$C_7$haloalkyl; X is oxygen or sulfur; $Y_1$, $Y_2$, $Y_3$, $Y_4$, $Y_5$ and $Y_6$ independently of one another are oxygen or sulfur; Z is oxygen or sulfur; n is 0; 1, 2, 3 or 4; and q is 1 or 2, which comprises

a)in the case of compounds of the formula I in which W is $W_1$, $W_4$, $W_5$, $W_6$ or $W_7$ and Q is -COO-, reacting a compound of the formula IIa

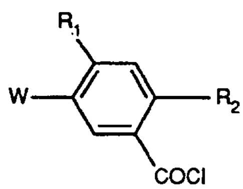 (IIa)

in which $R_1$ and $R_2$ are as defined in formula I and W is $W_1$, $W_4$, $W_5$, $W_6$ or $W_7$, with a compound of the formula IIIa

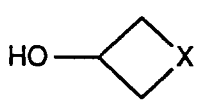 (IIIa)

in which X is as defined under formula I, in the presence of a base;

b) in the case of compounds of the formula I in which W is $W_1$, $W_4$, $W_5$, $W_6$ or $W_7$ and Q is oxygen or sulfur, reacting a compound of the formula IIb

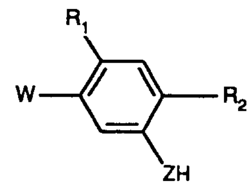
(IIb)

in which Z, $R_1$ and $R_2$ are as defined in formula I and W is $W_1$, $W_4$, $W_5$, $W_6$ or $W_7$, with a compound of the formula IIIb

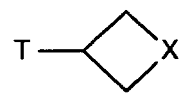
(IIIb)

in which X is as defined in formula I and T is tosyl or mesyl, preferably mesyl, in the presence of a base;

c) in the case of compounds of the formula I in which W is $W_1$, $W_4$, $W_5$, $W_6$ or $W_7$ and Q is -Z-CH-($R_3$)-COO-, reacting a compound of the formula IIc

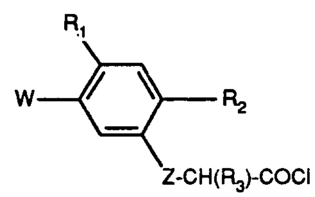
(IIc)

in which Z, $R_1$, $R_2$ and $R_3$ are as defined in formula I and W is $W_1$, $W_4$, $W_5$, $W_6$ or $W_7$, with a compound of the formula IIIa

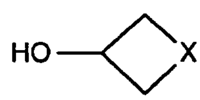
(IIIa)

in which X is as defined in formula I, in the presence of a base, or a compound of the formula IIb

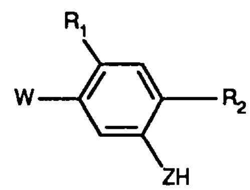
(IIb)

in which Z, $R_1$ and $R_2$ are as defined in formula I and W is $W_1$, $W_4$, $W_5$, $W_6$ and $W_7$, with a compound of the formula IIIc

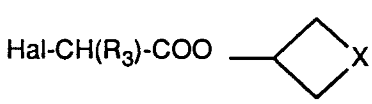
(IIIc)

in which X and $R_3$ are as defined for formula I and Hal is fluorine, chlorine or bromine, in the presence of a base;

d) in the case of compounds of the formula I in which in which W is $W_1$, $W_4$, $W_5$, $W_6$ or $W_7$ and Q is the group

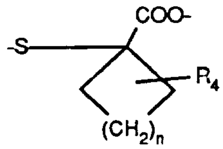

,

reacting a compound of the formula IId

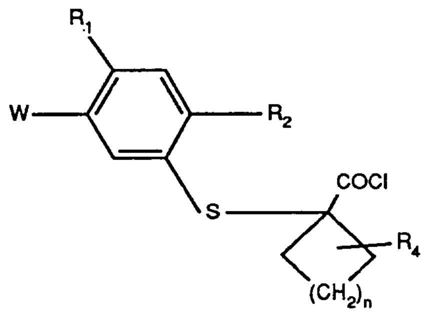

(IId)

in which $R_1$, $R_2$, $R_4$ and n are as defined in formula I and W is $W_1$, $W_4$, $W_5$, $W_6$ or $W_7$, with a compound of the formula IIIa

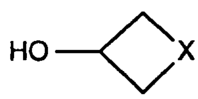

(IIIa)

in which X is as defined in formula I, in the presence of a base;

e) in the case of compounds of the formula I in which W is $W_1$, $W_4$, $W_5$, $W_6$ or $W_7$ and Q is the group CO-$Y_4$-CH($R_{10}$)-COO-, reacting a compound of the formula IIe

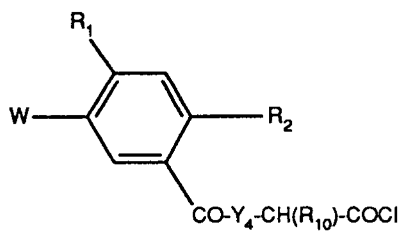

(IIe)

in which $R_1$, $R_2$, $R_3$ and $Y_4$ are as defined in formula I and W is $W_1$, $W_4$, $W_5$, $W_6$ or $W_7$, with a compound of the formula IIIa

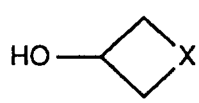

(IIIa)

in which X is as defined in formula I, in the presence of a base;

f) in the case of compounds of the formula I in which W is $W_2$ and $Y_1$ is sulfur, condensing an isothiocyanate of the formula XII

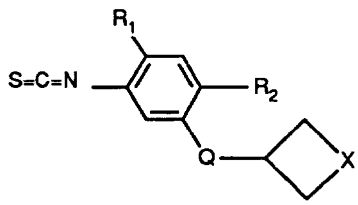

(XII)

in which $R_1$, $R_2$, X and Q are as defined in formula I, with a piperidine of the formula X

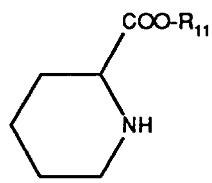

(X)

in which $R_{11}$ is $C_1$-$C_6$alkyl or benzyl, in an organic solvent in the presence of a base at a temperature from 0 to +150 ° C, to give the compound of the formula XIII

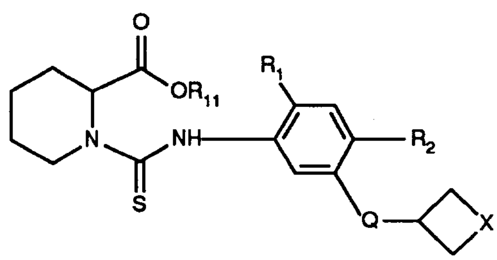

(XIII)

in which $R_1$, $R_2$, X and Q are as defined in formula I and $R_{11}$ is as defined in formula X, and the product is subsequently cyclised under acid conditions at a temperature of +50 to +150 ° C to give the compound of the formula I;

g) in the case of compounds of the formula I in which W is $W_3$ and $Y_2$ is sulfur, condensing an isocyanate of the formula IX

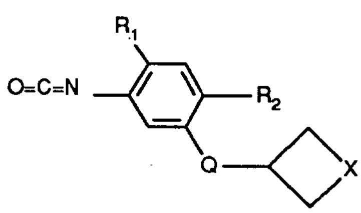

(IX)

in which $R_1$, $R_2$, Q and X are as defined in formula I, with a compound of the formula XIX

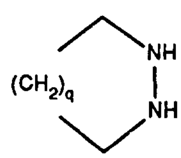

(XIX)

in which q is as defined in formula I, in an organic solvent in the presence of a base at a temperature from 0 to +150 ° C, and subsequently reacting the resulting compound of the formula

XXV

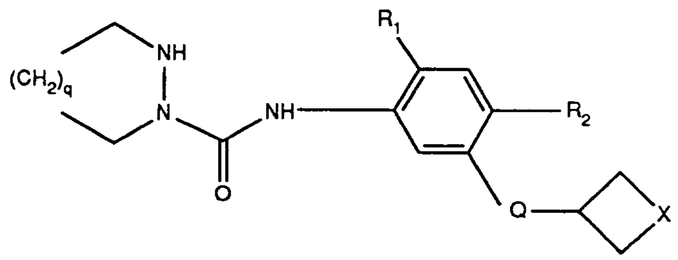

(XXV)

in which $R_1$, $R_2$, q, X and Q are as defined above, with thiophosgene in the presence of an inorganic base at a temperature of 0 to +150°C;

h) in the case of compounds of the formula I in which W is $W_3$ and $Y_2$ is oxygen, condensing an isocyanate of the formula IX

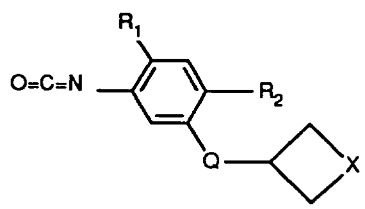

(IX)

in which $R_1$, $R_2$, X and Q are as defined above, with a compound of the formula XXVI

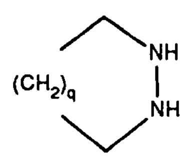

(XXVI)

in which q is as defined in formula I, in an organic solvent in the presence of a base at a temperature of 0 to +150°C, and subsequently reacting the resulting compound of the formula XXVII

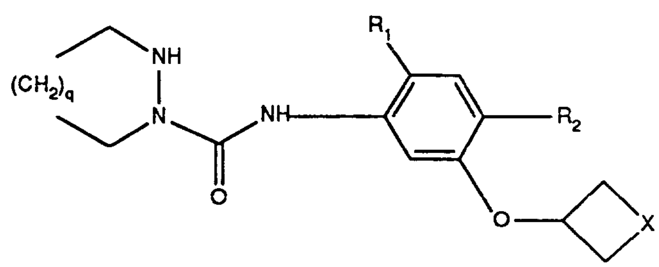

(XXVII)

in which $R_1$, $R_2$, q, X and Q are as defined above, with a chloroformic acid ester of the formula XXVIII

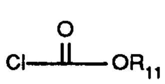

(XXVIII)

in which $R_{11}$ is $C_1$-$C_6$alkyl or benzyl, in the presence of a base at a temperature of 0 to +150°C, to give a compound of the formula XXIX

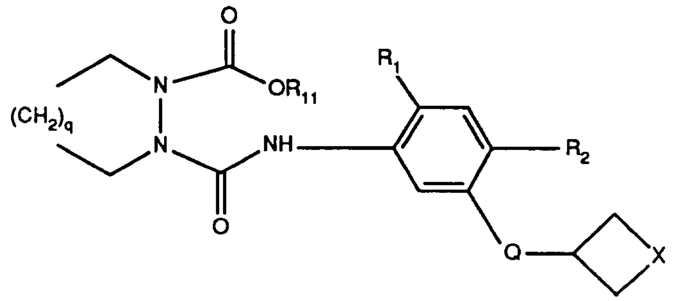

(XXIX)

in which $R_1$, $R_2$, $R_{11}$, q, X and Q are as defined above, and these compounds of the formula XXIX are cyclised in an inert solvent in the presence of a base at a temperature of +50 to +180 °C to give the compounds of the formulae IIa to IIe;

i) in the case of compounds of the formula I in which W is $W_8$, converting an isothiocyanate of the formula XII

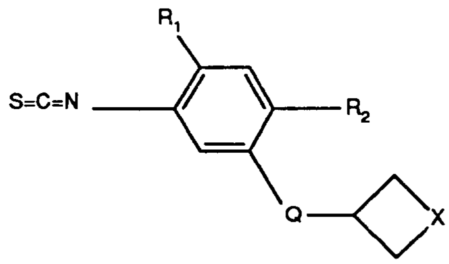

(XII)

in which $R_1$, $R_2$, X and Q are as defined in formula I, with a hexahydropyridazine of the formula XVIII

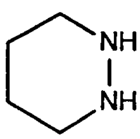

(XVIII)

into the compound of the formula XIX

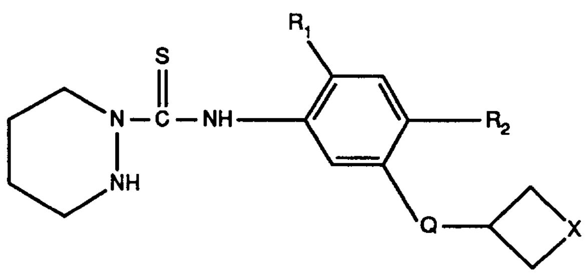

(XIX)

in which $R_1$, $R_2$, X and Q are as defined above, and subsequently reacting these compounds of the formula XIX with a compound of the formula XXX

$CY_3Cl_2$ (XXX),

in which $Y_3$ is oxygen or sulfur, in the presence of a base;

j) in the case of compounds of the formula I in which W is $W_9$, reacting a 2-hydroxycyclohex-1-enecarboxanilide or -thiocarboxanilide of the formula XXXI

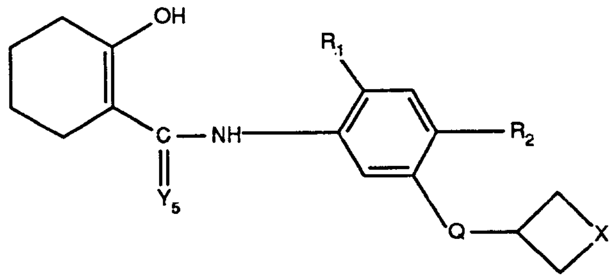

(XXXI)

in which Q, X, $R_1$, $R_2$ and $Y_5$ are as defined in formula I, with a reactive carbonic acid derivative or thiocarbonic acid derivative of the formula XXXII

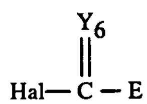

(XXXII)

in which $Y_6$ is oxygen or sulfur, Hal is chlorine or bromine and E is chlorine, bromine or $C_1$-$C_4$alkoxy, during which process cyclisation takes place to give the compound of the formula I.

**16.** A herbicidal and plant-growth-inhibiting composition, which comprises one or more oxetane and thiaetane derivatives of the formula I according to claim 1.

**17.** A composition according to claim 16, which comprises between 0.1% and 95% of active ingredient of the formula I according to claim 1.

**18.** A method of controlling undesired plant growth, which comprises applying an effective amount of an active ingredient of the formula I according to claim 1, or a composition according to claim 16 which comprises this active ingredient, to the plants or their environment.

**19.** The method according to claim 18, which comprises applying the active ingredient in an amount of between 0.001 and 2 kg per hectare.

**20.** The method according to claim 18 for the selective control of weeds in crops of useful plants, pre- or post-emergence.

**21.** A method for inhibiting the growth of plants, which comprises applying an effective amount of an active ingredient of the formula I according to claim 1, or a composition according to claim 16 which comprises this active ingredient, to the plants or their environment.

European Patent Office

# EUROPEAN SEARCH REPORT

Application Number

EP 92 11 4448

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 810 254 (F. HOFFMANN- LA ROCHE AG) * page 32, line 15-18 ; page 33, line 15-20 * | 1,16 | C07D405/12 A01N43/20 C07D409/12 C07D513/04 |
| Y | EP-A-0 415 642 (ROHM AND HAAS CY) * page 14, compound 4; page 17, compound 117, 118 ; page 18, compound 135 * | 1,16 | |
| Y | EP-A-0 083 055 (SUMITOMO CHEMICAL COMPANY, LTD.) * page 13, compound 12 * | 1,16 | |
| A | EP-A-0 364 797 (BAYER AG) * claims * | 1,16 | |
| A | EP-A-0 308 702 (BAYER AG) * claims * | 1,16 | |
| P,Y | EP-A-0 468 924 (CIBA-GEIGY AG) * claims * | 1,16 | |

TECHNICAL FIELDS SEARCHED (Int. Cl.5)

C07D

The present search report has been drawn up for all claims

| Place of search | Date of completion of the search | Examiner |
|---|---|---|
| THE HAGUE | 06 OCTOBER 1992 | VAN BIJLEN H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)